# EUROPEAN PATENT SPECIFICATION

(11) **EP 2 714 927 B1**
(45) Date of publication and mention of the grant of the patent: **10.08.2016**
(21) Application number: 12725624.6
(22) Date of filing: 01.06.2012
(51) Int. Cl.: C12Q 1/68

(54) **METHODS AND DEVICES FOR PROGNOSIS OF CANCER RELAPSE**
VERFAHREN UND VORRICHTUNGEN ZUR PROGNOSE EINES KREBSREZIDIVS
PROCÉDÉS ET DISPOSITIFS POUR LE PRONOSTIC D'UNE RECHUTE DU CANCER

(30) Priority: 01.06.2011 DK 201100416
(43) Date of publication of application: 09.04.2014
(73) Proprietor: Medical Prognosis Institute A/S, 2970 Horsholm (DK)
(72) Inventor: KNUDSEN, Steen, DK-3460 Birkroed (DK); MAZIN, Wiktor, DK-2840 Holte (DK)
(74) Representative: Lahrtz, Fritz
(86) International application number: PCT/EP2012/002332
(87) International publication number: WO 2012/163541

(56) References cited:
- WO-A2-2011/135459
- CN-A- 102 002 490
- CASTELLI E C ET AL: "In silico analysis of microRNAS targeting the HLA-G 3' untranslated region alleles and haplotypes", HUMAN IMMUNOLOGY, NEW YORK, NY, US, vol. 70, no. 12, 1 December 2009 (2009-12-01), pages 1020-1025, XP026750950, ISSN: 0198-8859, DOI: 10.1016/J.HUMIMM.2009.07.028 [retrieved on 2009-08-05]
- GALLARDO ELENA ET AL: "miR-34a as a prognostic marker of relapse in surgically resected non-small-cell lung cancer.", CARCINOGENESIS NOV 2009 LNKD- PUBMED:19736307, vol. 30, no. 11, November 2009 (2009-11), pages 1903-1909, XP002680754, ISSN: 1460-2180
- XU LING ET AL: "[Association of miRNAs expression profiles with prognosis and relapse in childhood acute lymphoblastic leukemia].", ZHONGHUA XUE YE XUE ZA ZHI = ZHONGHUA XUEYEXUE ZAZHI MAR 2011 LNKD- PUBMED:21535956, vol. 32, no. 3, March 2011 (2011-03), pages 178-181, XP009161486, ISSN: 0253-2727

### SEQUENCE LISTING

<110> Medical Prognosis Institute
<120> METHODS AND DEVICES FOR PROGNOSIS OF CANCER TREATMENTS AND USES THEREOF IN DIAGNOSIS
<130> 50495-004W01
<150> PA 2011 00416
   <151> 2011-06-01
<160> 4
<170> PatentIn version 3.5
<210> 1
   <211> 22
   <212> RNA
   <213> Artificial Sequence
<220>
   <223> hsa-miR-513b
<400> 1
   uucacaagga ggugucauuu au 22
<210> 2
   <211> 21
   <212> RNA
   <213> Artificial Sequence
<220>
   <223> hsa-miR-650
<400> 2
   aggaggcagc gcucucagga c 21
<210> 3
   <211> 20
   <212> RNA
   <213> Artificial Sequence
<220>
   <223> hsa-miR-324-3p
<400> 3
   acugccccag gugcugcugg 20
<210> 4
   <211> 22
   <212> RNA
   <213> Artificial Sequence
<220>
   <223> hsa-miR-1307
<400> 4
   acucggcgug gcgucggucg ug 22

## Description

### FIELD OF THE INVENTION

The invention features methods, devices, and kits for prognosing cancer relapse in a cancer patient.

### BACKGROUND OF THE INVENTION

Gene expression analysis in tumor samples from patients has been used to facilitate cancer prognosis and diagnosis. Gene expression patterns can reveal the presence of cancer in a patient, its type, stage, and origin, and whether genetic mutations are involved. Gene expression may even have a role in predicting the efficacy of chemotherapy.

In recent years a new class of regulatory molecules, microRNAs, has been discovered. Determining their concentration, or expression, in cancer cells has revealed a role in cancer. It has been demonstrated that the detection of microRNAs can be used to determine the site of origin of cancers and can be used to differentiate between aggressive and non-aggressive cancers. Information contained in the expression level of genes and microRNAs is complementary, and combining this information in methods of prognosis or diagnosis may produce results that are more clinically accurate and useful.

Lung cancer is a disease with high mortality. Even after surgery, the majority of lung cancer patients suffer a relapse and die. If the removed tumor is more than 3 cm in diameter, the standard of care is to offer the patient chemotherapy to prevent relapse. If the tumor is less than 3 cm in diameter, and no spreading of the tumor is observed (also referred to as Stage Ia), the patient is offered no further treatment. Yet more than half of lung cancer patients suffer a relapse and die within 5 years.

There is a need for methods for prognosing cancer relapse in a patient with a cancer after one or more medical treatments for cancer, including surgery.

### SUMMARY OF THE INVENTION

The invention includes a method for prognosing cancer relapse in a cancer patient before or after one or more cancer treatments (e.g., surgery, radiation therapy, and/or chemotherapy) by determining the level of expression of at least one biomarker (e.g., more than one biomarker, such as 2, 3, or 4 or more biomarkers), in which the biomarker has at least 85% (e.g., 85%, 90%, 95%, 97%, 99%, or 100%) sequence identity to the sequence of hsa-miR-513B, (SEQ ID NO: 1). In one embodiment, the method involves determining the expression level of a biomarker having the sequence of hsa-miR-650, ,singly or in any combination of 2, 3, or all 4 biomarkers (either simultaneously or in sequence).

In another embodiment, the methods of the invention may include determining the levels of expression of pair-wise combinations of the hsa-miR-650, with hsa-miR-324-3p, hsa-miR-513b, and hsa-miR-1307 biomarkers (or a biomarker having at least 85%, 90%, 95%, 97%, 99%, or 100% sequence identity to the sequence of any one of the hsa-miR-650, hsa-miR-324-3p, hsa-miR-513b, and hsa-miR-1307 biomarkers respectively). In another embodiment, the methods of the invention may include determining the levels of expression of triplet or quadruplet combinations of the hsa-miR-650, with hsa-miR-324-3p, hsa-miR-513b, and hsa-miR-1307 biomarkers (or a biomarker having at least 85%, 90%, 95%, 97%, 99%, or 100% sequence identity to the sequence of any one of the hsa-miR-650, hsa-miR-324-3p, hsa-miR-513b, and hsa-miR-1307 biomarkers respectively). The methods of the invention may include determining the level of expression of two or more biomarkers (e.g., 2, 3, or 4 biomarkers) simultaneously or in sequence.

The methods of the invention include determining the level of expression of the biomarker(s) in a sample from a cancer patient. The sample may be a blood sample or a tissue sample, e.g., a tumor sample. The methods of the invention can be used for prognosing relapse of any type of a non-small cell lung carcinoma, before or after a first cancer treatment in a cancer patient. In one embodiment of the invention, the methods of prognosing cancer relapse in a cancer patient may occur after a first cancer treatment. Alternatively, the prognosis may occur prior to a first cancer treatment. In another embodiment, the prognosis may occur after a first treatment but before a second treatment. Alternatively, the prognosis may occur after the second cancer treatment. The cancer treatment described in the invention may include one or more of surgery, radiation therapy, and chemotherapy and/or any other therapy known in the art for treating cancer. In one aspect of the invention, the chemotherapeutic agent may include one or more of a drug, an antibody, and an oligonucleotide.

In one aspect of the method, an increase or a decrease in the level of expression of at least one biomarker (e.g., a biomarker having at least 85% (e.g., 85%, 90%, 95%, 97%, 99%, or 100%) sequence identity to the sequence of hsa-miR-513b, (SEQ ID NO: 1)) indicates a good prognosis of no cancer relapse.

The methods of the invention may include prognosing cancer relapse based on level of expression of at least one biomarker (e.g., a biomarker having at least 85% (e.g., 85%, 90%, 95%, 97%, 99%, or 100%) sequence identity to the sequence of has -miR-513b, (SEQ ID NO: 1)) in a cancer patient sample relative to level of expression of the biomarker(s) in a sample from a normal patient, or from a sample from a patient after a first (or subsequent) cancer treatment. Alternatively, the detection of expression of one or more biomarkers would alone provide sufficient information for a cancer relapse prognosis.

The methods of the invention may include collecting nucleic acid molecules from a patient (e.g., cancer patient) sample (e.g., a tissue sample, such as a tumour sample) and, optionally, using a quantitative reverse transcription-polymerase chain reaction (qRT-PCR) to amplify the nucleic acid molecules, followed by detection of one or more biomarkers (e.g., 1, 2, 3, or 4 biomarkers) in the sample or determining the expression level of at least one biomarker (e.g., 1, 2, 3, or 4 biomarkers) in the sample.

The invention features devices that can be used to detect the expression of, or determine the expression level of, at least one biomarker (e.g., more than one biomarker, such as 2, 3, or 4 or more biomarkers) and may include at least one (e.g., more than one, such as 2, 3, or 4 or more) single-stranded nucleic acid molecule (also referred to as an oligonucleotide probe) having at least 85% (e.g., 85%, 90%, 95%, 97%, 99%, or 100%) sequence identity to the sequence of a biomarker or its complement sequence. The sequence of the biomarker includes at least 5 (e.g., 5, 6, 7, 8, 10, 12, 15, 20, or 22) consecutive nucleotides of the sequence of any one of hsa-miR-513b, (SEQ ID NOs: 1). For example, the devices may include oligonucleotide probes that can be used to detect the expression of hsa-miR-513b, biomarker (SEQ ID NOs: 1), or sequences complementary to this biomarker, in a tissue sample from a patient (e.g., a cancer patient).

In one embodiment, the device includes oligonucleotide probes having at least 100% sequence identity to the sequence of the hsa-miR-513b, biomarker, or the complement sequences. The device can include pair-wise, triple, or quadruple combinations of oligonucleotide probes having at least 85%, 90%, 95%, 97%, 99%, or 100% sequence identity to the sequence of the hsa-miR-513b, with hsa-miR-650, hsa-miR-324-3p, and hsa-miR-1307 biomarkers (SEQ ID NOs: 1-4, respectively), or their complement sequences.

The device allows specific hybridization between single stranded nucleic acid molecules of the device (e.g., oligonucleotide probes) and the biomarker(s) or its complement sequence(s). The device includes at least one single-stranded nucleic acid molecule having a length in the range of 10 to 100 nucleotides (e.g., a length of 10, 20, 25, 30, 40, 60, 80, or 100 nucleotides or a length in the range of 5-50, 20-50, or 20-100 nucleotides). When the device is contacted with a diverse population of nucleic acid molecules prepared from a sample under conditions that allow hybridisation, the oligonucleotide probes in the device hybridize with their target biomarker and can detect the presence of at least one biomarker (e.g., 1, 2, 3, or 4 biomarkers) in a sample (e.g., a patient tissue sample). Alternatively, the device can be used to determine the expression level of one or more of (e.g., 1, 2, 3, or 4) the above-mentioned biomarkers. In one embodiment of the invention, the device is a microarray device.

The invention includes methods for prognosing cancer relapse in a cancer patient by using the devices described above for detecting, or for determining the level of expression of, at least one biomarker (e.g., a biomarker having at least 85% (e.g., 85%, 90%, 95%, 97%, 99%, or 100%) sequence identity to the sequence of hsa-miR-513b, (SEQ ID NO: 1)) in a patient sample (e.g., a tumor sample), such that the detection of, or the level of expression of one or more (e.g., 1, 2, 3, or 4) biomarkers is prognostic of cancer relapse in the patient. The sample can be from a patient diagnosed with any one of the cancers described herein (e.g., a lung cancer, more specifically, non-small cell lung cancer). The device can be used for prognosis of cancer relapse in a cancer patient before or after a first cancer treatment. Alternatively, the device can be used for prognosis of cancer relapse after a first cancer treatment, but before a second treatment. In yet another aspect of the invention, the device can be used for prognosis of cancer relapse after a second cancer treatment.

The device of the method can be used to detect an increase or a decrease in the level of expression of at least one of the above-mentioned biomarkers (e.g., 1, 2, 3, or 4 biomarkers) indicating a good prognosis of no cancer relapse. Alternatively, the device can be used to detect an increase or a decrease in the level of expression of one or more of the above-mentioned biomarkers (e.g., 1, 2, 3, or 4 biomarkers) indicating a poor prognosis of cancer relapse.

The device can be used for prognosing cancer relapse based on level of expression of one or more biomarkers in a cancer patient sample relative to level of expression in a sample from a normal patient, or from a sample from a patient after a first cancer treatment. Alternatively, detection of expression of one or more biomarkers by the device can be used to provide a prognosis for cancer relapse.

The invention also features a kit that may include reagents for collecting nucleic acid molecules from a sample from a cancer patient, reagents for amplifying nucleic acid molecules collected from the sample to produce an amplified sample, and at least one device for detecting the level of expression of at least one biomarker (e.g., 1, 2, 3, or 4 biomarkers) having the sequence of any one of SEQ ID NO: 1 in the amplified sample. In one embodiment, a quantitative reverse transcription-polymerase chain reaction (qRT-PCR) may be used to produce the amplified sample. The kit may further include instructions for prognosing cancer relapse in a cancer patient based on the level of expression of the at least one biomarker.

In one embodiment, the kit may include the device described above (e.g., a microarray device) to detect at least one (e.g., 1, 2, 3, or 4) biomarker (e.g., a biomarker having the sequence of SEQ ID NO: 1) in the sample or to determine the expression level of at least one (e.g., 1, 2, 3, or 4) biomarkers in the sample. The kit may further include instructions for applying nucleic acid molecules collected from the sample to the device, and/or instructions for detecting hybridization of at least one oligonucleotide probe with at least one biomarker or its complement sequence in order to detect the expression of, or to determine the expression level of the at least one biomarker in the sample. The kit may further include instructions for prognosing cancer relapse in a cancer patient based on the level of expression of the at least one biomarker as detected using the device.

Biomarkers relevant for prognosing cancer relapse are identified as those that are differentially expressed between the relevant groups for which prognosis is warranted. For example, samples obtained from cancer patients may be assayed for the biomarkers of the invention to group patients according to whether or not the patient experiences a relapse after a cancer treatment e.g., surgery, radiation therapy, and/or chemotherapy. Total RNA, including mRNA and microRNA is extracted from the samples and labeled according to standard procedures. The amount of mRNA from each known gene, or microRNA from each microRNA species known, is measured with one or more DNA microarrays containing probes complementary to the mRNAs and/or microRNAs.

This approach can be used for mRNA biomarkers, as well as for microRNA biomarkers. Prognosis is based on mRNA biomarkers, microRNA biomarkers, or combinations thereof, all measured using one or more DNA microarrays (or RT-PCR) on labeled RNA extracted from a sample from the patient's tumor.

The method of the invention can be applied for prognosis of cancer (e.g., lung cancer) relapse prior to or after treatment. There is currently no good and accurate method for determining whether a cancer will relapse or not. The methods described herein can be used by, e.g., an oncologist, to choose the most appropriate treatment for the patient based on the genetic makeup of the individual tumor. Knowing the likelihood of relapse will allow the oncologist to select one or more appropriate chemotherapy regimens, or a combination of surgery, chemotherapy, and radiation therapy.

"Cancer patient" as used herein refers to a subject, e.g., a human subject, who has, or has had a cancer and may or may not have been treated for the cancer.

"Complement" of a nucleic acid sequence or a "complementary" nucleic acid sequence as used herein refers to an oligonucleotide which is in "antiparallel association" when it is aligned with the nucleic acid sequence such that the 5' end of one sequence is paired with the 3' end of the other. Nucleotides and other bases may have complements and may be present in complementary nucleic acids. Bases not commonly found in natural nucleic acids that may be included in the nucleic acids of the present invention include, for example, inosine and 7-deazaguanine. "Complementarity" may not be perfect; stable duplexes of complementary nucleic acids may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

When complementary nucleic acid sequences form a stable duplex, they are said to be "hybridized" or to "hybridize" to each other or it is said that "hybridization" has occurred. Nucleic acids are referred to as being "complementary" if they contain nucleotides or nucleotide homologues that can form hydrogen bonds according to Watson-Crick base-pairing rules (e.g., G with C, A with T or A with U) or other hydrogen bonding motifs such as for example diaminopurine with T, 5-methyl C with G, 2-thiothymidine with A, inosine with C, pseudoisocytosine with G, etc. Anti-sense RNA may be complementary to other oligonucleotides, e.g., mRNA.

"Biomarker" as used herein indicates a gene or other portion of a subject's genetic material that is expressed in a form that can be measured (e.g., as an mRNA, microRNA, or protein) and whose expression indicates good or poor prognosis of cancer relapse in a patient.

"Marker gene" or "biomarker gene" as used herein means a gene in a cell the expression of which correlates to sensitivity or resistance of the cell (and thus the patient from which the cell was obtained) to a treatment (e.g., exposure to a compound).

"Microarray" as used herein means a device employed by any method that quantifies one or more subject oligonucleotides, e.g., DNA or RNA, or analogues thereof, at a time. One exemplary class of microarrays consists of DNA probes attached to a glass or quartz surface. For example, many microarrays, including those made by Affymetrix, use several probes for determining the expression of a single gene. The DNA microarray may contain oligonucleotide probes that may be, e.g., full-length cDNAs complementary to an RNA or cDNA fragments that hybridize to part of an RNA. The DNA microarray may also contain modified versions of DNA or RNA, such as locked nucleic acids or LNA. Exemplary RNAs include mRNA, miRNA, and miRNA precursors. Exemplary microarrays also include a "nucleic acid microarray" having a substrate-bound plurality of nucleic acids, hybridization to each of the plurality of bound nucleic acids being separately detectable. The substrate may be solid or porous, planar or non-planar, unitary or distributed. Exemplary nucleic acid microarrays include all of the devices so called in Schena (ed.), DNA Microarrays: A Practical Approach (Practical Approach Series), Oxford University Press (1999); Nature Genet. 21(1)(suppl.):1-60 (1999); Schena (ed.), Microarray Biochip: Tools and Technology, Eaton Publishing Company/BioTechniques Books Division (2000). Additionally, exemplary nucleic acid microarrays include substrate-bound plurality of nucleic acids in which the plurality of nucleic acids are disposed on a plurality of beads, rather than on a unitary planar substrate, as is described, inter alia, in Brenner et al., Proc. Natl. Acad. Sci. USA 97(4):1665-1670 (2000). Examples of nucleic acid microarrays may be found in U.S. Pat. Nos. 6,391,623, 6,383,754, 6,383,749, 6,380,377, 6,379,897, 6,376,191, 6,372,431, 6,351,712 6,344,316, 6,316,193,6,312,906, 6,309,828, 6,309,824, 6,306,643, 6,300,063, 6,287,850, 6,284,497, 6,284,465, 6,280,954, 6,262,216, 6,251,601, 6,245,518, 6,263,287, 6,251,601, 6,238,866, 6,228,575, 6,214,587, 6,203,989, 6,171,797, 6,103,474, 6,083,726, 6,054,274, 6,040,138, 6,083,726, 6,004,755, 6,001,309, 5,958,342, 5,952,180, 5,936,731, 5,843,655, 5,814,454, 5,837,196, 5,436,327, 5,412,087, 5,405,783.

Exemplary microarrays may also include "peptide microarrays" or "protein microarrays" having a substrate-bound plurality of polypeptides, the binding of a oligonucleotide, a peptide, or a protein to each of the plurality of bound polypeptides being separately detectable. Alternatively, the peptide microarray, may have a plurality of binders, including but not limited to monoclonal antibodies, polyclonal antibodies, phage display binders, yeast 2 hybrid binders, aptamers, which can specifically detect the binding of specific oligonucleotides, peptides, or proteins. Examples of peptide arrays may be found in WO 02/31463, WO 02/25288, WO 01/94946, WO 01/88162, WO 01/68671, WO 01/57259, WO 00/61806, WO 00/54046, WO 00/47774, WO 99/40434, WO 99/39210, WO 97/42507 and U.S. Pat. Nos. 6,268,210, 5,766,960, 5,143,854.

"Gene expression" as used herein means the amount of a gene product in a cell, tissue, organism, or subject, e.g., amounts of DNA, RNA, or proteins, amounts of modifications of DNA, RNA, or protein, such as splicing, phosphorylation, acetylation, or methylation, or amounts of activity of DNA, RNA, or proteins associated with a given gene.

"Treatment" or "medical treatment" means administering to a subject or living organism or exposing to a cell or tumor a compound (e.g., a drug, a protein, an antibody, an oligonucleotide, a chemotherapeutic agent, and a radioactive agent) or some other form of medical intervention used to treat or prevent cancer (e.g., lung cancer) or the symptoms of cancer (e.g., cryotherapy and radiation therapy). Radiation therapy includes the administration to a patient of radiation generated from sources such as particle accelerators and related medical devices that emit X-radiation, gamma radiation, or electron (beta radiation) beams. A treatment may further include surgery, e.g., to remove a tumor from a subject or living organism.

Other features and advantages of the invention will be apparent from the following Detailed Description, the drawings, and the claims.

### BRIEF DESCRIPTION OF THE DRAWINGS

Figure 1 is a graph showing a Kaplan-Meier plot of recurrence in 78 non-small cell lung carcinoma (NSCLC) patients predicted in a leave-one-out cross-validation using a 60-microRNA model.
Figure 2 is a graph showing a Kaplan-Meier plot of overall survival in 30 NSCLC patients predicted in an independent validation using a 4-microRNA model.

### DETAILED DESCRIPTION

The invention features methods for determining the expression level of one or more biomarkers from a patient sample for prognosing cancer relapse in a cancer patient before or after a cancer treatment (e.g., surgery and/or treatment with one or more, and preferably two or more, chemotherapeutic agents and/or radiation). The invention also features devices (e.g., a microarray) that include nucleic acid probes that can detect the expression of one or more biomarkers from a patient sample. The devices can be used for prognosing whether a cancer in a patient will relapse before or after a treatment. The invention also features kits to determine the level of expression of one or more biomarkers from a patient sample for prognosing cancer relapse in a cancer patient. The methods according to the present invention can be implemented using software that is run on an apparatus for measuring gene expression in connection with a detection device, such as a microarray. The detection device (e.g., a microarray, such as a DNA microarray), which is included in a kit for processing a tumor sample from a subject, and the apparatus for reading the device and turning the result into a prognosis for the subject, may be used to implement the methods of the invention.

### Cancers

The methods, devices, and kits of the disclosure can be used for prognosing cancer relapse in a patient suffering from, or diagnosed with, cancer, for example, a cancer of the breast, prostate, lung (e.g., non small cell lung carcinoma), bronchus, colon, rectum, urinary bladder, skin, kidney, pancreas, oral cavity, pharynx, ovary, thyroid, parathyroid, stomach, brain, esophagus, liver, intrahepatic bile duct, cervix larynx, heart, testis, small intestine, large intestine, anus, anal canal, anorectum, vulva, gallbladder, pleura, bone, joint, hypopharynx, eye and/or orbit, nose, nasal cavity, middle ear, nasopharynx, ureter, peritoneum, omentum, mesentery, and/or gastrointestines, as well as any form of cancer including, e.g., chronic myeloid leukemia, acute lymphocytic leukemia, non-Hodgkin lymphoma, melanoma, carcinoma, basal cell carcinoma, malignant mesothelioma, neuroblastoma, multiple myeloma, leukemia, retinoblastoma, acute myeloid leukemia, chronic lymphocytic leukemia, Hodgkin lymphoma, carcinoid tumors, acute tumor, and/or soft tissue sarcoma (e.g., preferably the cancer is a cancer of the bladder, breast, colon, rectum, uterus, kidney, lung, skin (e.g., melanoma), pancreas, prostate, blood and/or bone marrow (e.g., leukemia), lymphocytes (e.g., non-Hodgkin lymphoma), and/or thyroid).

### Cancer Treatments

The methods, devices, and kits of the invention can be used to determine the potential for relapse of a cancer in a cancer patient, e.g., a lung cancer patient, before or after a first treatment. The first treatment may include, e.g., one or more of surgery, radiation therapy, and/or chemotherapy. The chemotherapy may include administration of one or more of (e.g., two or more of) the following agents: vincristine, cisplatin, etoposide, azaguanine, carboplatin, adriamycin, aclarubicin, mitoxantrone, mitoxantrone, mitomycin, paclitaxel, gemcitabine, taxotere, dexamethasone, ara-c, methylprednisolone, methotrexate, bleomycin, methyl-gag, belinostat, carboplatin, 5-fu (5-fluorouracil), idarubicin, melphalan, IL4-PR38, valproic acid, all-trans retinoic acid (ATRA), cytoxan, topotecan, suberoylanilide hydroxamic acid (SAHA, vorinostat), depsipeptide (FR901228), bortezomib, leukeran, fludarabine, vinblastine, busulfan, dacarbazine, oxaliplatin, hydroxyurea, tegafur, daunorubicin, estramustine, mechlorethamine, streptozocin, carmustine, lomustine, mercaptopurine, teniposide, dactinomycin, tretinoin, ifosfamide, tamoxifen, irinotecan, floxuridine, thioguanine, PSC 833, erlotinib, herceptin, bevacizumab, celecoxib, fulvestrant, iressa, anastrozole, letrozole, cetuximab, rituximab, radiation, histone deacetylase (HDAC) inhibitors, and 5-Aza-2'-deoxycytidine (decitabine).

A beneficial aspect of the invention is that the methods, devices, and kits can be used for prognosing cancer relapse in a cancer patient before or after one or more treatments for cancer (e.g., two, three, four, five, ten, twenty, thirty, or more treatments for cancer) by assaying the level of expression of one or more (e.g., two, three, or four) biomarker hsa-miR-513b, either simultaneously or in sequence. The expression of each of these biomarkers has been determined to be prognostic for cancer relapse in a patient. Other biomarkers that can be used for prognosing cancer relapse in a patient include one or more of the biomarkers listed in Tables 1 and 2 below.

The methods, devices, and kits of the disclosure can also be used to identify patient subpopulations that are responsive to one or more treatments thought to be ineffective for treating disease (e.g., cancer) in the general population. More generally, prognosis of cancer relapse in a cancer patient treated with one or more treatments can be done using biomarker expression regardless of knowledge about patient's prior cancer treatments. The methods of the present invention can be implemented using software that is run on an apparatus for measuring gene expression in connection with a microarray. Devices of the disclosure (e.g., a microarray, such as a DNA and/or RNA microarray) can be included in a kit for processing a tumor sample from a subject (e.g., a cell, tissue, or organ sample containing a tumor or a cell thereof), and the apparatus for reading the device and turning the result into a prognosis profile for the subject may be used to implement the methods of the invention.

### Biomarkers of the Invention

The invention features biomarkers having at least 85% (e.g., 85%, 90%, 95%, 97%, 99%, or 100%) sequence identity to the sequence of any one of hsa-miR-513b (5' UUCACAAGGAGGUGUCAUUUAU3'; SEQ ID NO:1); hsa-miR-650 (5' AGGAGGCAGCGCUCUCAGGAC3'; SEQ ID NO: 2); hsa-miR-324-3p (5' ACUGCCCCAGGUGCUGCUGG3'; SEQ ID NO: 3); and hsa-miR-1307 (ACUCGGCGUGGCGUCGGUCGUG; SEQ ID NO 4). Additional biomarkers that can be used in the methods, devices, and kits of the invention are listed in Tables 1 and 2 below. These additional biomarkers can be used in combination with the biomarkers having 85% or more sequence identity (e.g., 100% sequence identity) to the sequence of hsa-miR-513b. Preferably, the biomarkers of the invention have the sequence of hsa-miR-650, hsa-miR-324-3p, hsa-miR-513b, and hsa-miR-1307 and may be used in any combination with has-miR-513b (simultaneously or in sequence) as described below. Furthermore any combination of these four biomarkers may be used with one or more of biomarkers listed in Tables 1 and 2 (simultaneously or in sequence).

The biomarkers of the methods may be used in methods, devices and kits, as described below, to determine the potential for relapse of a cancer (e.g., lung cancer) in a patient before or after one or more treatments for cancer, such as the cancer treatments listed above.

### Methods for prognosing cancer relapse in a cancer patient using the biomarkers of the invention

The invention features methods for prognosing cancer relapse in a patient with a cancer before or after one or more treatments for cancer, e.g., surgery, radiation therapy, and/or chemotherapy, by measuring the level of expression of one or more (e.g., 1, 2, 3, or 4) biomarkers having at least 85% (e.g., 85%, 90%, 95%, 97%, 99%, or 100%) sequence identity to the sequence of hsa-miR-513b. Preferably, the method involves determining the expression level of a biomarker having the sequence of hsa-miR-650, (SEQ ID NO: 1), either singly or in any combination of 2, 3, or all 4 (either simultaneously or in sequence). Preferably, the method is performed in a patient after at least one treatment for cancer.

For example, the methods of the invention may include determining the levels of expression of pair-wise combinations of has-miR-513b with the hsa-miR-650, hsa-miR-324-3p, and hsa-miR-1307 biomarkers (or a biomarker having at least 85%, 90%, 95%, 97%, 99%, or 100% sequence identity to the sequence of any one of the hsa-miR-650, hsa-miR-324-3p, hsa-miR-513b, and hsa-miR-1307 biomarkers). In particular, the levels of expression of the following pair-wise combinations of biomarkers can be measured, either simultaneously or in sequence:
1) hsa-miR-513b and hsa-miR-650;
2) hsa-miR-513b and hsa-miR-324-3p;
3) hsa-miR-513b and hsa-miR-1307;
4) hsa-miR-650 and hsa-miR-513b;
5) hsa-miR-324-3p and hsa-miR-513b;
6) hsa-miR-1307 and hsa-miR-513b;

The methods of the invention may also include determining the levels of expression of triple combinations of the hsa-miR-513B, biomarker (or a biomarker having at least 85%, 90%, 95%, 97%, 99%, or 100% sequence identity to the sequence of hsa-miR-513b. In particular, the levels of expression of the following triple combinations of biomarkers can be measured, either simultaneously or in sequence:
1) hsa-miR-513b, hsa-miR-650, hsa-miR-324-3p;
2) hsa-miR-513b, hsa-miR-650, hsa-miR-1307;
3) hsa-miR-513b, hsa-miR-324-3p, hsa-miR-650;
4) hsa-miR-513b, hsa-miR-324-3p, hsa-miR-1307;
5) hsa-miR-513b, hsa-miR-1307, hsa-miR-650;
6) hsa-miR-513b, hsa-miR-1307, hsa-miR-324-3p;
7) hsa-miR-650, hsa-miR-513b, hsa-miR-324-3p;
8) hsa-miR-650, hsa-miR-513b, hsa-miR-1307;
9) hsa-miR-650, hsa-miR-1307, hsa-miR-513b;
10) hsa-miR- 324-3p, hsa-miR-513b, hsa-miR-650;
11) hsa-miR-324-3p, hsa-miR-513b, hsa-miR-1307;
12) hsa-miR-324-3p, hsa-miR-1307, hsa-miR-513b;
13) hsa-miR-1307, hsa-miR-513b, hsa-miR-650;
14) hsa-miR-1307, hsa-miR-513b, hsa-miR-324-3p;
15) hsa-miR-1307, hsa-miR-650, hsa-miR-513b;
16) hsa-miR-1307, hsa-miR-324-3p, hsa-miR-513b; and

The methods of the invention may also include determining the levels of expression of quadruple combinations of hsa-miR-513b, biomarker (or a biomarker having at least 85%, 90%, 95%, 97%, 99%, or 100% sequence identity to the sequence of the hsa-miR-513b. In particular, the levels of expression of the following quadruple combinations of biomarkers can be determined, either simultaneously or in sequence:
1) hsa-miR-513b, hsa-miR-650, hsa-miR-324-3p, hsa-miR-1307;
2) hsa-miR-513b, hsa-miR-650, hsa-miR-1307, hsa-miR-324-3p;
3) hsa-miR-513b, hsa-miR-324-3p, hsa-miR-650, hsa-miR-1307;
4) hsa-miR-513b, hsa-miR-324-3p, hsa-miR-1307, hsa-miR-650;
5) hsa-miR-513b, hsa-miR-1307, hsa-miR-650, hsa-miR-324-3p;
6) hsa-miR-513b, hsa-miR-1307, hsa-miR-324-3p, hsa-miR-650;
7) hsa-miR-650, hsa-miR-513b, hsa-miR-324-3p, hsa-miR-1307;
8) hsa-miR-650, hsa-miR-513b, hsa-miR-1307, hsa-miR-324-3p;
9) hsa-miR-650, hsa-miR-324-3p, hsa-miR-513b, hsa-miR-1307;
10) hsa-miR-650, hsa-miR-324-3p, hsa-miR-1307, hsa-miR-513b;
11) hsa-miR-650, hsa-miR-1307, hsa-miR-513b, hsa-miR-324-3p;
12) hsa-miR-650, hsa-miR-1307, hsa-miR-324-3p, hsa-miR-513b;
13) hsa=miR-324-3p, hsa-miR-513b, hsa-miR-650, hsa-miR-1307;
14) hsa-miR-324-3p, hsa-miR-513b, hsa-miR-1307, hsa-miR-1307;
15) hsa-miR-324-3p, hsa-miR-650, hsa-miR-513b, hsa-miR-1307;
16) hsa-miR-324-3p, hsa-miR-650, hsa-miR-1307, hsa-miR-513b;
17) hsa-miR-324-3p, hsa-miR-1307, hsa-miR-513b, hsa-miR-650;
18) hsa-miR-324-3p, hsa-miR-1307, hsa-miR-650, hsa-miR-513b;
19) hsa-miR-1307, hsa-miR-513b, hsa-miR-650, hsa-miR-324-3p;
20) hsa-miR-1307, hsa-miR-513b, hsa-miR-324-3p, hsa-miR-650;
21) hsa-miR-1307, hsa-miR-650, hsa-miR-513b, hsa-miR-324-3p;
22) hsa-miR-1307, hsa-miR-650, hsa-miR-324-3p, hsa-miR-513b;
23) hsa-miR-1307, hsa-miR-324-3p, hsa-miR-513b, hsa-miR-650; and
24) hsa-miR-1307, hsa-miR-324-3p, hsa-miR-650m, hsa-miR-513b.

The methods of the invention may include collecting nucleic acid samples from a sample, e.g., a tissue sample. The sample is preferably a tumor sample from a cancer patient. For example, the sample may be from a lung cancer patient, such as a patient suffering from a non-small cell lung carcinoma. The methods of the invention may optionally include amplifying the nucleic acid molecules using, e.g., polymerase chain reaction (PCR), to produce an amplified solution. The methods of the invention may further include performing qRT-PCR in a thermal cycler using the nucleic acid molecules collected from a sample or using the amplified solution described above to measure the level of expression of one or more of the biomarkers in the sample. Procedures for performing qRT-PCR are described in, e.g., U.S. Patent No. 7,101,663 and U.S. Patent Application Nos. 2006/0177837 and 2006/0088856. The level of expression of two or more of the hsa-miR-650, hsa-miR-324-3p, hsa-miR-513b, and hsa-miR-1307 biomarkers (and, optionally, one or more additional biomarkers listed in Tables 1 and 2) in the sample can be determined simultaneously in the same reaction. Alternatively, the level of expression of two or more of the hsa-miR-650, hsa-miR-324-3p, hsa-miR-513b, and hsa-miR-1307 biomarkers (and, optionally, one or more additional biomarkers listed in Tables 1 and 2, if desired) in the sample can be determined simultaneously in different reactions. Furthermore, the level of expression of two or more of the hsa-miR-650, hsa-miR-324-3p, hsa-miR-513b, and hsa-miR-1307 biomarkers (as well as one or more additional biomarkers listed in Tables 1 and 2, if desired) can be determined one after the other in the same or separate reactions.

The methods of the invention may also include prognosing cancer relapse in a cancer patient after one or more cancer treatments, e.g., surgery, radiation therapy, and/or chemotherapy, based on the level of expression of hsa-miR-513b, biomarker (and, optionally, one or more additional biomarkers listed in Tables 1 and 2, if desired) in the sample.

For example, an increase in the level of expression of one or more of the biomarkers may indicate a good prognosis of no relapse after one or more cancer treatments, such as those treatments described above. Alternatively, a decrease in the level of expression of one or more of the biomarkers may indicate a good prognosis of no relapse after one or more cancer treatments, such as those described above. Furthermore, an increase in the level of expression of one or more of the biomarkers may indicate a poor prognosis of cancer relapse after one or more cancer treatments. Alternatively, a decrease in the level of expression of one or more of the biomarkers may indicate a poor prognosis of cancer relapse after one or more cancer treatments. Alternatively the detection of expression alone of any of the biomarkers may be an indication of the prognosis of the relapse of a cancer in a cancer patient after a cancer treatment.

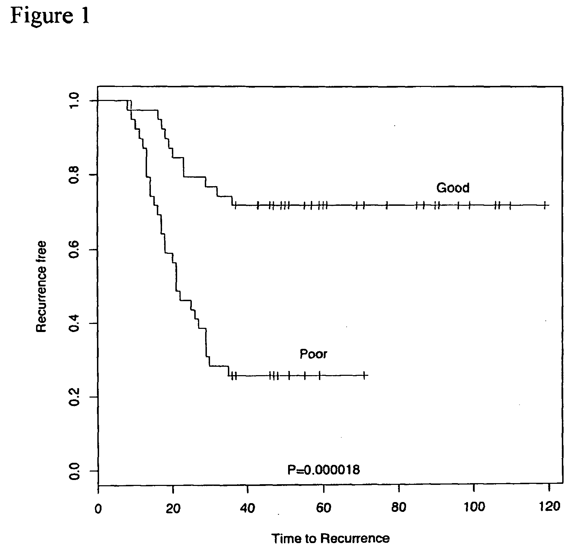

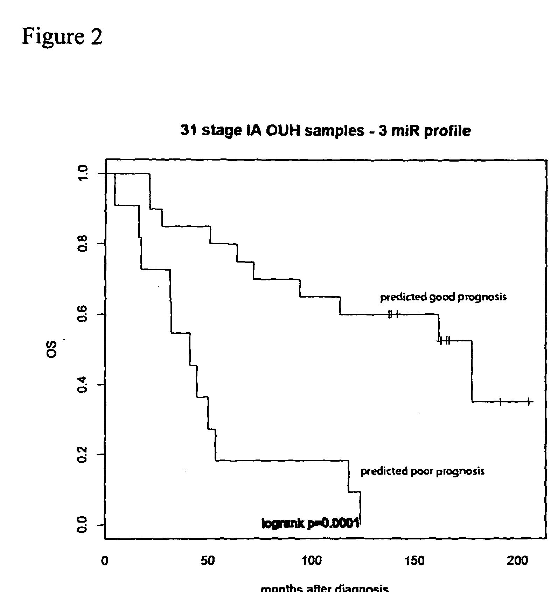

A good prognosis refers to a case where the patient will be alive at least 5 years (e.g., 4, 5, 6, 7, 8, 10, or 12 or more years) after a first cancer treatment, and a poor prognosis refers to a case where the patient will not likely survive for at least 5 years after a first cancer treatment. Kaplan-Meier curves can be used to compare survival over time, as shown in figures 1 and 2.

In the methods for prognosing cancer relapse, the expression level of one or more of the biomarkers can be determined relative to that in a normal cell or relative to a cancer cell from a patient who has undergone a first course of treatment.

### Devices and methods for cancer prognosis using biomarkers of the invention

The invention features devices that include one or more oligonucleotide probes having a sequence that is identical, or complementary, to at least 5 (e.g., 5, 6, 7, 8, 10, 12, 15, 20, or 22; preferably 22) consecutive nucleotides (or nucleotide analogues) of the sequence of hsa-miR-513b, biomarker. The oligonucleotide probes of the devices may also include sequences having at least 85% (e.g., 85%, 90%, 95%, 97%, 99%, or 100%) sequence identity to the sequence of any one of the hsa-miR-513b, hsa-miR-650, hsa-miR-324-3p, and hsa-miR-1307 biomarkers (SEQ ID NOs: 1-4, respectively), or their complements, over at least about 5 (e.g., 5, 6, 7, 8, 10, 12, 15, 20, or 22; preferably 22) consecutive nucleotides (or nucleotide analogues). For example, the devices may include oligonucleotide probes that can be used to detect the presence of, or the level of expression of, any one or more (e.g., any combination of) the hsa-miR-513b, hsa-miR-650, hsa-miR-324-3p, and hsa-miR-1307 biomarkers (SEQ ID NOs: 1-4, respectively), or sequences complementary to these biomarkers, in a tissue sample from a patient.

Preferably, a device of the invention includes oligonucleotide probes having a sequence with at least 85% sequence identity to the sequence of one or more of the hsa-miR-513b, hsa-miR-650, hsa-miR-324-3p, and hsa-miR-1307 biomarkers (SEQ ID NOs: 1-4, respectively), or their complements, over at least about 22 consecutive nucleotides (or nucleotide analogues). More preferably, the device includes oligonucleotide probes having at least 100% sequence identity to the sequence of any one or more of the hsa-miR-513b, hsa-miR-650, hsa-miR-324-3p, and hsa-miR-1307 biomarkers, or their complements. The devices may include probes that can be used to detect the presence, or level of expression, of only one of the biomarkers, or they may include probes that can be used to detect the presence, or level of expression, of combinations of 2, 3, or 4 of the biomarkers.

For example, the device can include the following pair-wise combinations of oligonucleotide probes having at least 85%, 90%, 95%, 97%, 99%, or 100% sequence identity to the sequence of any one of the hsa-miR-513b, hsa-miR-650, hsa-miR-324-3p, and hsa-miR-1307 biomarkers (SEQ ID NOs: 1-4, respectively), or their complements, over at least about 5 (e.g., 5, 6, 7, 8, 10, 12, 15, 20, or 22; preferably 22) consecutive nucleotides (or nucleotide analogues):
1) hsa-miR-513b and hsa-miR-650;
2) hsa-miR-513b and hsa-miR-324-3p;
3) hsa-miR-513b and hsa-miR-1307;

Preferably, the device includes pair-wise combinations of oligonucleotide probes that have at least 85% sequence identity to the sequence of the hsa-miR-513b with, hsa-miR-650, hsa-miR-324-3p, and hsa-miR-1307 biomarkers (SEQ ID NOs: 1-4, respectively), or their complements, over at least about 22 consecutive nucleotides (or nucleotide analogues). More preferably, the device includes pair-wise combinations of oligonucleotide probes that have at least 100% sequence identity to the sequence of the hsa-miR-513b with, hsa-miR-650, hsa-miR-324-3p, and hsa-miR-1307 biomarkers (SEQ ID NOs: 1-4, respectively), or their complements, over at least about 22 consecutive nucleotides (or nucleotide analogues).

The device can also include the following triplet combinations of oligonucleotide probes having at least 85%, 90%, 95%, 97%, 99%, or 100% sequence identity to the sequence of the hsa-miR-513b with, hsa-miR-650, hsa-miR-324-3p, and hsa-miR-1307 biomarkers (SEQ ID NOs: 1-4, respectively), or their complements, over at least about 5 (e.g., 5, 6, 7, 8, 10, 12, 15, 20, or 22) consecutive nucleotides (or nucleotide analogues):
1) hsa-miR-513b, hsa-miR-650, hsa-miR-324-3p;
2) hsa-miR-513b, hsa-miR-650, hsa-miR-1307;
3) hsa-miR-513b, hsa-miR-324-3p, hsa-miR-1307; and

Preferably, the device includes triplet combinations of oligonucleotide probes that have at least 85% sequence identity to the sequence of the hsa-miR-513b with, hsa-miR-650, hsa-miR-324-3p, and hsa-miR-1307 biomarkers (SEQ ID NOs: 1-4, respectively), or their complements, over at least about 22 consecutive nucleotides (or nucleotide analogues). More preferably, the device includes triplet combinations of oligonucleotide probes that have at least 100% sequence identity to the sequence of the hsa-miR-513b with, hsa-miR-650, hsa-miR-324-3p, and hsa-miR-1307 biomarkers (SEQ ID NOs: 1-4, respectively), or their complements, over at least about 22 consecutive nucleotides (or nucleotide analogues).

The device can also include oligonucleotide probes having at least 85%, 90%, 95%, 97%, 99%, or 100% sequence identity to the sequence of each of the hsa-miR-513b, hsa-miR-650, hsa-miR-324-3p, and hsa-miR-1307 biomarkers (SEQ ID NOs: 1-4, respectively), or their complements, over at least about 5 (e.g., 5, 6, 7, 8, 10, 12, 15, 20, or 22) consecutive nucleotides (or nucleotide analogues). Preferably, the device includes oligonucleotide probes that have at least 85% sequence identity to the sequence of each of the hsa-miR-513b, hsa-miR-650, hsa-miR-324-3p, and hsa-miR-1307 biomarkers (SEQ ID NOs: 1-4, respectively), or their complements, over at least about 22 consecutive nucleotides (or nucleotide analogues). More preferably, the device includes oligonucleotide probes that have at least 100% sequence identity to the sequence of each of the hsa-miR-513b, hsa-miR-650, hsa-miR-324-3p, and hsa-miR-1307 biomarkers (SEQ ID NOs: 1-4, respectively), or their complements, over at least about 22 consecutive nucleotides (or nucleotide analogues).

The oligonucleotide probes of the devices described above may have a length of, e.g., 5-20, 25, 5-50, 5-100, 25-100, 50-100, or over 100 nucleotides. The oligonucleotide probes may be deoxyribonucleic acids (DNA) or ribonucleic acids (RNA).

The invention also features methods of using the devices described above to detect the expression of or determine the level of expression of hsa-miR-513b, biomarker, or any combination of two or more of the has-miR-513bwith the hsa-miR-650, hsa-miR-324-3p, , and hsa-miR-1307 biomarkers, in a patient sample for prognosing cancer relapse after a cancer treatment.

The device of the disclosure containing one or more oligonucleotide probes can be a microarray device. The microarray device may contain oligonucleotide probes that may be, e.g., cDNAs corresponding to or complementary to an RNA (e.g., an mRNA) or a microRNA, or the oligonucleotide probes may be cDNA fragments that hybridize to part of an RNA (e.g., an mRNA) or a microRNA. Exemplary RNAs include miRNA, and miRNA precursors. Exemplary microarrays also include a "nucleic acid microarray" having a substrate-bound plurality of nucleic acids, hybridization to each of the plurality of bound nucleic acids being separately detectable.

The microarrays of the disclosure can include one or more oligonucleotide probes that have nucleotide sequences that are identical to or complementary to, e.g., at least 5, 8, 12, 20, 22, 30, 40, 60, 80, 100, 150, or 200 consecutive nucleotides (or nucleotide analogues) of the hsa-miR-650, hsa-miR-324-3p, hsa-miR-513b, and hsa-miR-1307 biomarkers and/or to one or more of the biomarkers listed in Tables 1 and 2 below. The oligonucleotide probes may have a length in the range of, e.g., 5-20, 5-50, 25-50, 5-100, 25-100, 50-100, or over 100 nucleotides long. The oligonucleotide probes may be deoxyribonucleic acids (DNA) or ribonucleic acids (RNA) or analogues thereof, such as LNA. Consecutive nucleotides within the oligonucleotide probes (e.g., 5-20, 25, 5-50, 50-100, or over 100 consecutive nucleotides), which are used as biomarkers of responsiveness to a cancer treatment, may also appear as consecutive nucleotides in one or more of the genes described herein beginning at or near, e.g., the first, tenth, twentieth, thirtieth, fortieth, fiftieth, sixtieth, seventieth, eightieth, ninetieth, hundredth, hundred-fiftieth, two-hundredth, five-hundredth, or one-thousandth nucleotide of the genes listed in Tables 1 and 2 below.

When a diverse population of nucleic acid molecules prepared from a sample, e.g., a patient sample is applied to the devices described above, the target nucleic acid molecule(s) in the sample hybridizes with the probe(s) on the device. This hybridization allows the detection of, and/or a determination of the quantity of, a target nucleic acid molecule(s) in the sample (e.g., one or more of the biomarkers described herein), and provides a readout of the level of expression of that target nucleic acid molecule(s). The target nucleic acid molecule(s) may be any one of the hsa-miR-513b, hsa-miR-650, hsa-miR-324-3p, and hsa-miR-1307 biomarkers described above, any may also include any one or more of the biomarkers described in Tables 1 and 2 below. The devices described above may be used to simultaneously (or sequentially) detect, or determine the level of expression of, one or more of these biomarkers. Optionally, the nucleic acid molecules isolated from the sample may be amplified prior to detection using the device of the invention using, e.g., PCR, to produce an amplified sample. The amplified sample can then be applied to a device of the invention.

The devices of the invention can be used in methods to determine the expression level of the hsa-miR-513b, biomarker in a sample for prognosing cancer relapse in a cancer patient before and/or after one or more cancer treatments. The devices can be used to simultaneously (or sequentially) determine the expression level of multiple biomarkers, for example, 2, 3, or 4 biomarkers, and to use this information to determine a for cancer relapse prognosis for a patient.

In one example, cell/tissue samples are snap frozen in liquid nitrogen until processing. RNA may be extracted using, e.g., Trizol Reagent from Invitrogen following the manufacturer's instructions. RNA can be amplified using, e.g., MessageAmp kit from Ambion Inc. following the manufacturer's instructions. MicroRNA can be extracted from formalin-fixed paraffin embedded samples using, e.g., RecoverAll (Ambion Inc.) and labeled using, e.g., Genisphere HSR (GenisPhere Inc.). Amplified RNA can be quantified using, e.g., the HG-U 133A GeneChip from Affymetrix Inc and a compatible apparatus, e.g., the GCS3000Dx from Affymetrix, using the manufacturer's instructions. MicroRNA can be quantified using Affymetrix miRNA version 1.0 or 2.0.

The resulting gene expression measurements can be further processed for example, as described in examples 1-3. The procedures described can be implemented using R software available from R-Project and supplemented with packages available from Bioconductor.

For lung cancer prognosis hsa-miR-513b, biomarker may be sufficient to give an accurate prediction. Preferably two or more of the hsa-miR-513b with, hsa-miR-650, hsa-miR-324-3p, and hsa-miR-1307 biomarkers are used. In addition, 3 to 50 mRNA or microRNA biomarkers, such as those listed in Tables 1 and 2, can be used to provide an even more accurate prediction. Given the relatively small number of biomarkers required, procedures such as quantitative reverse transcriptase polymerase chain reaction (qRT-PCR) may be performed to determine, with greater precision, the amount of biomarkers expressed in a sample. This will provide an alternative to or a complement to the use of devices described above. For example, qRT-PCR may be performed alone or in combination with a microarray described herein.

### Kits for prognosing cancer relapse after a cancer treatment in cancer patients

The invention also features kits for prognosing cancer relapse in a cancer patient (e.g., a lung cancer patient) after one or more cancer treatments. The kits may include reagents for collecting nucleic acid molecules from a sample from a patient. For example, the kits may include reagents for lysis of patient samples and/or for isolating and purifying RNA from patient samples. The kits may further include reagents for amplifying the nucleic acid molecules isolated from the patient sample, for example, by PCR. The kits may include reagents for determining the level of expression of one or more biomarkers having at least 85% (e.g., 85%, 90%, 95%, 97%, 99%, or 100%) sequence identity to the sequence of any one of the hsa-miR-650, hsa-miR-324-3p, hsa-miR-513b, and hsa-miR-1307 biomarkers, or their complements, using assays known in the art, e.g., qRT-PCR. The kits may include primers and probes for performing qRT-PCR to determine the expression level of the biomarkers described above. The kits may include instructions prognosing cancer relapse based on the level of expression of one or more biomarkers determined using the kits.

The kits may further include any one of the devices described above, to which a nucleic acid sample from a patient or an amplified solution may be applied, so that the probes on the device can hybridize with target biomarkers in the sample and provide a readout of the level of expression of one or more biomarkers (e.g., one or more of the hsa-miR-513b, hsa-miR-650, hsa-miR-324-3p, and hsa-miR-1307 biomarkers) in the sample. The device allows the simultaneous (or sequential) measurement of the level of expression of one or more of the biomarkers in a sample. The device in the kits may be a microarray device. The kits may further include instructions for prognosing cancer relapse in a cancer patient, e.g., a good prognosis or a poor prognosis, based on the level of expression of one or more the biomarkers determined using the devices described above. Additionally, the device of the kits can be used in combination with qRT-PCR based assays to determine the level of expression of one or more the biomarkers. Furthermore, the kits may include software programs for prognosing cancer relapse based on the expression level of the biomarkers.

In one example, the kits may include reagents for RNA extraction from tumors (e.g., Trizol from Invitrogen Inc), reagents for RNA amplification (e.g., MessageAmp from Ambion Inc), a microarray for determining gene expression (e.g., the HG-U 133A GeneChip from Affymetrix Inc), a microarray hybridization station and scanner (e.g., the GeneChip System 3000Dx from Affymetrix Inc), and software for analyzing the expression levels of biomarkers, as described herein (e.g., implemented in R from R-Project or S-Plus from Insightful Corp.).

### EXAMPLES

### Example 1: MicroRNAs useful for lung cancer prognosis

Formalin fixed paraffin embedded (FFPE) tissue specimens from 79 patients with pathologic stage 1 NSCLC were used for analysis. Clinical data was collected from Roswell Park Cancer Institute's tumor registry and was validated by chart review. Tissue was deparaffinized and miRNA extracted. After quality control assessments of the extracted RNA, hybridization was performed to a locked nucleic acid (LNA) based array platform (Exiqon Inc.) containing probes for all miRs in miRBase version 11. Data from the arrays was background corrected and Loess normalized. In a leave-one-out cross validation, miRNAs differentially expressed between patients with recurrence and patients without, were selected with a t-test, using a multiple testing correction leaving a false discovery rate of 0.1%. The resulting miRNAs were subjected to Principal Component Analysis and the five most important components used to train a multivariate classifier using classification algorithms K nearest neighbor, nearest centroid, neural network and support vector machine. The left out sample was predicted by majority vote among the classification algorithms into Good or Poor prognosis. A Kaplan-Meier plot was prepared of the time to recurrence for the Good and Poor prognosis groups. A log-rank test for statistical significance of the difference between the two groups was performed.
**RESULTS** - Of 79 samples, 78 samples passed the quality control conditions for hybridization. Data analysis performed as detailed above led to 60 microRNAs being selected for all 78 classifiers. The 60 microRNAs are shown in Table 1 together with a total of 157 microRNAs that are statistically significant (FDR=1%) in an analysis of all 78 samples and for which P-value and log2 fold change is calculated. The 60-gene model predicted outcome in a statistically significant fashion (Figure 1).

### Example 2: MicroRNAs useful for lung cancer prognosis

It is possible to use as few as 2 or 3 microRNAs to obtain classification of lung cancer samples as described in Example 1. If the 2 or 3 microRNAs are selected from the list of hsa-miR-141 hsa-miR-22 hsa-miR-200b* hsa-miR-630 hsa-miR-27a hsa-miR-510 hsa-miR-30c-1*, classification performance similar to that shown in Figure 1 can be achieved. The First Principal Component with a cutoff of 0 can be used alone to predict recurrence or non-recurrence. Other classification methods based on the expression of 2 or 3 microRNAs selected from Table 1 can be used as well.

### Example 3: Using Affymetrix arrays with DNA probes complementary to microRNAs.

Examples 1 and 2 involved the use of a locked nucleic acids platform from Exiqon to identify microRNAs that can be used to distinguish between patients with a good and a poor prognosis. A DNA-based platform such as Affymetrix has different physical and chemical properties, and will result in a different list of optimal microRNAs for the same purpose. The same FFPE samples used for Example 1 were analyzed on the Affymetrix *GeneChip*® *miRNA 1.0* array. Normalization was performed using constant totalRNA for each sample. A support vector machine svm from the library e1071 from www.bioconductor.org with default parameters was used to train a predictor. In cross-validation experiments, the following 3 microRNA probes on the Affy platform were best in separating poor prognosis from good prognosis patients: hsa- hsa-miR-650, hsa-miR-324-3p, hsa-miR-513b. Of these, hsa-miR-513b contributes most to the prognosis, followed by hsa-miR-650, followed by hsa-miR-324-3p, which is least important. If a fourth miR is desired, hsa-miR-1307 can be used and may improve performance on some datasets.

### Example 4: Independent validation of 4-microRNA profile from example 3.

The 3-microRNA profile from Example 3 was independently verified on a cohort of 31 NSCLC patients in Stage Ia (Figure 2). This cohort was normalized in the same manner as the cohort in example 3. Using the support vector machine trained on the cohort from Example 3, the patients were predicted with either good prognosis or poor prognosis. The Kaplan-Meier curve in Figure 2 shows the overall survival in the two prediction groups. There is a statistically significant difference in survival between the good and poor prognosis groups (P=0.0001 in a logrank test).

**TABLE 1. List of probe IDs referring to the miRNAs on miRCURY LNA arrays (v. 10.0, Exiqon). 60-gene refers to whether or not the miRNA was selected for all classifiers in leave-one-out cross-validation. A value of TRUE means that the miRNA is more reliable and important.**

| ID | name | log2(FC) | P-value | 60-gene |
|---|---|---|---|---|
| 17327 | hsa-miR-630 | -0.421 | 2.74e-09 | TRUE |
| 17859 | hsa-miR-200b* | -0.438 | 8.78e-09 | TRUE |
| 42834 | hsa-miR-219-2-3p | -0.606 | 1.11e-07 | FALSE |
| 42682 | hsa-miR-25 | 0.979 | 1.47e-07 | FALSE |
| 42957 | hsa-miR-323-3p | -0.536 | 2.99e-07 | FALSE |
| 42702 | hsa-miR-30c-1* | -0.426 | 3.22e-07 | TRUE |
| 42593 | hsa-miR-623 | -0.551 | 3.23e-07 | TRUE |
| 5250 | hsa-miR-105 | 1.14 | 3.75e-07 | FALSE |
| 42524 | hsa-miR-21* | 0.77 | 1.46e-06 | FALSE |
| 17752 | hsa-let-7f | 1.13 | 1.5e-06 | FALSE |
| 10986 | hsa-miR-193a-3p | 1.1 | 2.03e-06 | FALSE |
| 42811 | hsa-miR-542-5p | -0.539 | 2.45e-06 | TRUE |
| 33596 | hsa-miR-126* | 1.17 | 2.48e-06 | FALSE |
| 19593 | hsa-miR-27a | 1.28 | 2.51e-06 | TRUE |
| 27720 | hsa-miR-15a | 1.23 | 2.82e-06 | FALSE |
| 30687 | hsa-miR-93 | 1.14 | 2.85e-06 | FALSE |
| 11065 | hsa-miR-335 | 1.17 | 3.29e-06 | FALSE |
| 11142 | hsa-miR-510 | -0.325 | 3.36e-06 | TRUE |
| 42458 | hcmv-miR-US25-1* | -0.51 | 3.59e-06 | TRUE |
| 14302 | hsa-miR-374b | 1.01 | 4.21e-06 | FALSE |
| 27537 | ebv-miR-BART13 | -0.432 | 4.5e-06 | TRUE |
| 13132 | hsa-miR-519e* | -0.325 | 5.12e-06 | TRUE |
| 27378 | hsa-miR-374a | 1.23 | 5.17e-06 | FALSE |
| 10985 | hsa-miR-191 | 1.16 | 5.35e-06 | TRUE |
| 10995 | hsa-miR-199a-3p/ hsa-miR-199b-3p | 1.26 | 5.37e-06 | FALSE |
| 10138 | hsa-miR-130a | 1.13 | 5.92e-06 | FALSE |
| 11078 | hsa-miR-365 | 0.661 | 6.44e-06 | FALSE |
| 27551 | hsa-miR-612 | -0.325 | 6.58e-06 | TRUE |
| 13143 | hsa-miR-301a | 1.11 | 7.03e-06 | FALSE |
| 17552 | hsa-miR-617 | -0.433 | 7.07e-06 | TRUE |
| 11022 | hsa-miR-221 | 0.931 | 8.4e-06 | FALSE |
| 17836 | hsa-miR-30b* | -0.392 | 8.52e-06 | TRUE |
| 10972 | hsa-miR-181b | 0.596 | 9.57e-06 | FALSE |
| 42513 | hsa-miR-300 | -0.339 | 1.16e-05 | TRUE |
| 42533 | hiv1-miR-H1 | -0.408 | 1.16e-05 | FALSE |
| 29562 | hsa-miR-199a-5p | 1.22 | 1.18e-05 | TRUE |
| 27541 | hcmv-miR-UL70-3p | -0.517 | 1.18e-05 | FALSE |
| 13175 | hsa-miR-27b | 1.26 | 1.2e-05 | TRUE |
| 42838 | miRPlus_42838 | -0.387 | 1.28e-05 | TRUE |
| 10998 | hsa-miR-19b | 1.34 | 1.42e-05 | FALSE |
| 10967 | hsa-miR-16 | 1.35 | 1.62e-05 | TRUE |
| 11020 | hsa-miR-22 | 1.03 | 1.74e-05 | TRUE |
| 10306 | hsa-miR-146b-5p | 1.02 | 1.75e-05 | FALSE |
| 42467 | hsa-miR-129-5p | -0.485 | 1.88e-05 | TRUE |
| 42843 | hsa-miR-654-5p | -0.411 | 2.11e-05 | TRUE |
| 42865 | hsa-miR-181a | 0.795 | 2.11e-05 | TRUE |
| 4610 | hsa-miR-126 | 1.08 | 2.11e-05 | TRUE |
| 4700 | hsa-miR-140-5p | 0.923 | 2.17e-05 | FALSE |
| 11023 | hsa-miR-222 | 0.881 | 2.21e-05 | TRUE |
| 19011 | hsa_SNORD10 | 0.903 | 2.22e-05 | TRUE |
| 17541 | ebv-miR-BART1-5p | -0.268 | 2.84e-05 | FALSE |
| 5740 | hsa-miR-21 | 1.5 | 2.91e-05 | TRUE |
| 19015 | hsa-miR-142-5p | 1.14 | 3.03e-05 | FALSE |
| 11182 | hsa-miR-98 | 0.837 | 3.12e-05 | FALSE |
| 11151 | hsa-miR-516b | -0.265 | 3.17e-05 | TRUE |
| 17608 | hsa-miR-425 | 0.753 | 3.35e-05 | FALSE |
| 17460 | hsa-miR-657 | -0.359 | 3.48e-05 | TRUE |
| 19580 | hsa-let-7i | 1.03 | 3.63e-05 | TRUE |
| 10997 | hsa-miR-19a | 1.35 | 3.67e-05 | FALSE |
| 28191 | hsa-miR-30e | 1.13 | 3.71e-05 | FALSE |
| 11104 | hsa-miR-422a | 0.423 | 3.78e-05 | FALSE |
| 42717 | hsa-miR-92b* | -0.447 | 3.94e-05 | TRUE |
| 27565 | hsa-miR-423-5p | -0.238 | 4.03e-05 | TRUE |
| 42929 | hsa-miR-25* | -0.279 | 4.33e-05 | TRUE |
| 17445 | hsa-miR-610 | -0.352 | 4.94e-05 | TRUE |
| 11279 | U6-snRNA-2 | 0.587 | 5.54e-05 | TRUE |
| 42532 | hsa-miR-22* | 0.455 | 5.73e-05 | FALSE |
| 19005 | hsa_SNORD118 | 0.665 | 5.91e-05 | TRUE |
| 42738 | hsa-miR-340* | -0.397 | 6.04e-05 | TRUE |
| 19602 | hsa-let-7g | 0.859 | 6.81e-05 | FALSE |
| 42831 | hsa-miR-28-5p | 0.953 | 7.32e-05 | FALSE |
| 31026 | hsa-miR-101 | 1.01 | 7.48e-05 | FALSE |
| 19591 | hsa-miR-199b-5p | 1.04 | 7.51e-05 | FALSE |
| 42758 | hsa-miR-640 | -0.389 | 7.78e-05 | TRUE |
| 29460 | hsa-miR-553 | -0.249 | 7.94e-05 | FALSE |
| 17328 | ebv-miR-BART8* | -0.465 | 7.99e-05 | TRUE |
| 42744 | hsa-miR-23a | 1.25 | 8.62e-05 | TRUE |
| 11040 | hsa-miR-29b | 1.17 | 8.91e-05 | FALSE |
| 42832 | hsa-miR-638 | -0.381 | 9.56e-05 | TRUE |
| 42570 | hsa-miR-194* | -0.448 | 9.65e-05 | TRUE |
| 19604 | hsa_SNORD4A | 0.728 | 0.000105 | TRUE |
| 42795 | kshv-miR-K12-3 | -0.326 | 0.000108 | TRUE |
| 10962 | hsa-miR-154 | 0.719 | 0.000127 | FALSE |
| 42902 | hsa-miR-185 | 0.375 | 0.000129 | TRUE |
| 42754 | hsa-miR-586 | -0.31 | 0.000135 | TRUE |
| 42887 | hsa-miR-331-3p | 0.473 | 0.000139 | TRUE |
| 17561 | ebv-miR-BART6-3p | -0.452 | 0.00014 | TRUE |
| 19585 | hsa-miR-148b | 0.757 | 0.000146 | FALSE |
| 17567 | kshv-miR-K12-1 | -0.206 | 0.00015 | FALSE |
| 42650 | hsa-miR-17 | 1.13 | 0.000153 | TRUE |
| 32884 | hsa-miR-342-3p | 0.974 | 0.000162 | FALSE |
| 17358 | ebv-miR-BART16 | -0.345 | 0.000164 | TRUE |
| 19582 | hsa-miR-106b | 0.96 | 0.000167 | TRUE |
| 42652 | hsa-miR-584 | -0.438 | 0.000178 | TRUE |
| 42802 | hsa-miR-150 | -0.236 | 0.000187 | TRUE |
| 10928 | hsa-miR-125a-5p | 0.531 | 0.000189 | TRUE |
| 33430 | hsa-miR-548b-3p | -0.307 | 0.000191 | TRUE |
| 42739 | hsa-miR-339-5p | 0.533 | 0.000192 | TRUE |
| 13485 | hsa-miR-10a | 0.882 | 0.000195 | FALSE |
| 13148 | hsa-miR-195 | 0.892 | 2e-04 | FALSE |
| 11030 | hsa-miR-26a | 1.19 | 0.000203 | FALSE |
| 42693 | hsa-miR-326 | -0.414 | 0.000209 | TRUE |
| 10946 | hsa-miR-141 | 1.08 | 0.000209 | TRUE |
| 17646 | ebv-miR-BHRF1-3 | -0.222 | 0.000211 | TRUE |
| 42648 | hsa-miR-106a | 1.16 | 0.000213 | TRUE |
| 42564 | hsa-miR-26b | 1.18 | 0.000237 | FALSE |
| 10925 | hsa-miR-10b | 0.781 | 0.00025 | FALSE |
| 42700 | hsa-miR-631 | -0.532 | 0.000254 | FALSE |
| 11024 | hsa-miR-223 | 0.727 | 0.000273 | FALSE |
| 19581 | hsa-miR-100 | 0.796 | 0.000273 | FALSE |
| 17280 | hsa-miR-15b | 1.06 | 0.000291 | FALSE |
| 42442 | hsa-miR-498 | -0.252 | 0.000325 | FALSE |
| 19008 | hsa_SNORD2 | 0.48 | 0.000357 | FALSE |
| 27533 | hsa-miR-320a | 0.543 | 0.00036 | FALSE |
| 10919 | hsa-miR-103 | 0.427 | 0.000361 | FALSE |
| 42528 | hsa-miR-296-3p | -0.335 | 0.000372 | FALSE |
| 42609 | hsa-miR-135a* | -0.295 | 0.000373 | FALSE |
| 42951 | ebv-miR-BHRF1-2 | -0.345 | 0.000398 | FALSE |
| 17506 | hsa-miR-24 | 1.26 | 0.000411 | FALSE |
| 17718 | hsa-miR-92b | 0.547 | 0.000425 | FALSE |
| 29872 | hsa-miR-340 | 0.338 | 0.000431 | FALSE |
| 28431 | miRPlus_28431 | -0.167 | 0.000436 | FALSE |
| 11053 | hsa-miR-32 | 0.881 | 0.000448 | FALSE |
| 42603 | hsa-miR-424* | -0.291 | 0.00045 | FALSE |
| 42965 | hsa-miR-424 | 0.629 | 0.000518 | FALSE |
| 42529 | hsa-miR-939 | -0.229 | 0.00053 | FALSE |
| 19606 | hsa_SNORD12 | 0.264 | 0.000534 | FALSE |
| 17952 | miRPlus_17952 | -0.231 | 0.000534 | FALSE |
| 42630 | hsa-miR-140-3p | 0.744 | 0.00056 | FALSE |
| 11027 | hsa-miR-23b | 1.14 | 0.000573 | FALSE |
| 42640 | hsa-miR-20b | 1.01 | 0.000582 | FALSE |
| 42649 | hsa-miR-20a | 0.995 | 0.000596 | FALSE |
| 11048 | hsa-miR-30a | 0.83 | 0.000605 | FALSE |
| 42679 | hsa-miR-642 | -0.29 | 0.000615 | FALSE |
| 42527 | hsa-miR-935 | -0.447 | 0.000622 | FALSE |
| 11134 | hsa-miR-502-5p | -0.152 | 0.000651 | FALSE |
| 17613 | hsa-miR-645 | -0.346 | 0.000655 | FALSE |
| 42751 | hsa-miR-720 | 0.501 | 0.000717 | FALSE |
| 11224 | hsa-miR-30e* | 0.687 | 0.000725 | FALSE |
| 17822 | hsa-miR-490-5p | -0.363 | 0.000729 | FALSE |
| 42695 | hsa-miR-596 | -0.36 | 0.000743 | FALSE |
| 42486 | hsa-miR-149* | -0.238 | 0.000744 | FALSE |
| 10978 | hsa-miR-184 | -0.21 | 0.000749 | FALSE |
| 11041 | hsa-miR-29c | 0.858 | 0.000763 | FALSE |
| 42782 | hcmv-miR-UL148D | -0.303 | 0.00078 | FALSE |
| 10947 | hsa-miR-142-3p | 0.962 | 0.000794 | FALSE |
| 28302 | miRPlus_28302 | 0.387 | 0.000857 | FALSE |
| 42573 | hsa-miR-1 | 0.323 | 0.000871 | FALSE |
| 42899 | hsa-miR-377* | -0.233 | 0.000896 | FALSE |
| 42845 | hsa-miR-125b-2* | -0.206 | 0.00091 | FALSE |
| 17463 | hsa-miR-151-3p | 0.597 | 0.000956 | FALSE |
| 30787 | hsa-miR-125b | 0.753 | 0.000967 | FALSE |
| 17470 | kshv-miR-K12-2 | -0.399 | 0.001 | FALSE |
| 42812 | hsa-miR-508-5p | -0.272 | 0.00106 | FALSE |
| 17493 | hsa-miR-622 | -0.358 | 0.0011 | FALSE |
| 42853 | hsa-miR-433 | -0.358 | 0.00116 | FALSE |
| 11175 | hsa-miR-525-5p | -0.188 | 0.00116 | FALSE |

**Table 2, The sequences of the mature microRNAs listed in Table 1**

| |
|---|
| hsa-let-7f UGAGGUAGUAGAUUGUAUAGUU |
| hsa-miR-15a UAGCAGCACAUAAUGGUUUGUG |
| hsa-miR-16 UAGCAGCACGUAAAUAUUGGCG |
| hsa-miR-17 CAAAGUGCUUACAGUGCAGGUAG |
| hsa-miR-19a UGUGCAAAUCUAUGCAAAACUGA |
| hsa-miR-19b UGUGCAAAUCCAUGCAAAACUGA |
| hsa-miR-20a UAAAGUGCUUAUAGUGCAGGUAG |
| hsa-miR-21 UAGCUUAUCAGACUGAUGUUGA |
| hsa-miR-22 AAGCUGCCAGUUGAAGAACUGU |
| hsa-miR-23a AUCACAUUGCCAGGGAUUUCC |
| hsa-miR-24 UGGCUCAGUUCAGCAGGAACAG |
| hsa-miR-25 CAUUGCACUUGUCUCGGUCUGA |
| hsa-miR-26a UUCAAGUAAUCCAGGAUAGGCU |
| hsa-miR-26b UUCAAGUAAUUCAGGAUAGGU |
| hsa-miR-27a UUCACAGUGGCUAAGUUCCGC |
| hsa-miR-28-5p AAGGAGCUCACAGUCUAUUGAG |
| hsa-miR-30a UGUAAACAUCCUCGACUGGAAG |
| hsa-miR-32 UAUUGCACAUUACUAAGUUGCA |
| hsa-miR-93 CAAAGUGCUGUUCGUGCAGGUAG |
| hsa-miR-98 UGAGGUAGUAAGUUGUAUUGUU |
| hsa-miR-100 AACCCGUAGAUCCGAACUUGUG |
| hsa-miR-101 UACAGUACUGUGAUAACUGAA |
| hsa-miR-29b UAGCACCAUUUGAAAUCAGUGUU |
| hsa-miR-103 AGCAGCAUUGUACAGGGCUAUGA |
| hsa-miR-105 UCAAAUGCUCAGACUCCUGUGGU |
| hsa-miR-106a AAAAGUGCUUACAGUGCAGGUAG |
| hsa-miR-199a-5p CCCAGUGUUCAGACUACCUGUUC |
| hsa-miR-129-5p CUUUUUGCGGUCUGGGCUUGC |
| hsa-miR-10a UACCCUGUAGAUCCGAAUUUGUG |
| hsa-miR-10b UACCCUGUAGAACCGAAUUUGUG |
| hsa-miR-181a AACAUUCAACGCUGUCGGUGAGU |
| hsa-miR-181b AACAUUCAUUGCUGUCGGUGGGU |
| hsa-miR-199b-5p CCCAGUGUUUAGACUAUCUGUUC |
| hsa-miR-221 AGCUACAUUGUCUGCUGGGUUUC |
| hsa-miR-222 AGCUACAUCUGGCUACUGGGU |
| hsa-miR-223 UGUCAGUUUGUCAAAUACCCCA |
| hsa-let-7g UGAGGUAGUAGUUUGUACAGUU |
| hsa-let-7i UGAGGUAGUAGUUUGUGCUGUU |
| hsa-miR-1 UGGAAUGUAAAGAAGUAUGUAU |
| hsa-miR-15b UAGCAGCACAUCAUGGUUUACA |
| hsa-miR-23b AUCACAUUGCCAGGGAUUACC |
| hsa-miR-27b UUCACAGUGGCUAAGUUCUGC |
| hsa-miR-125b UCCCUGAGACCCUAACUUGUGA |
| hsa-miR-130a CAGUGCAAUGUUAAAAGGGCAU |
| hsa-miR-140-5p CAGUGGUUUUACCCUAUGGUAG |
| hsa-miR-140-3p UACCACAGGGUAGAACCACGG |
| hsa-miR-141 UAACACUGUCUGGUAAAGAUGG |
| hsa-miR-142-3p UGUAGUGUUUCCUACUUUAUGGA |
| hsa-miR-142-3p UGUAGUGUUUCCUACUUUAUGGA |
| hsa-miR-191 CAACGGAAUCCCAAAAGCAGCUG |
| hsa-miR-125a-5p UCCCUGAGACCCUUUAACCUGUGA |
| hsa-miR-126 UCGUACCGUGAGUAAUAAUGCG |
| hsa-miR-150 UCUCCCAACCCUUGUACCAGUG |
| hsa-miR-154 UAGGUUAUCCGUGUUGCCUUCG |
| hsa-miR-184 UGGACGGAGAACUGAUAAGGGU |
| hsa-miR-185 UGGAGAGAAAGGCAGUUCCUGA |
| hsa-miR-193a-3p AACUGGCCUACAAAGUCCCAGU |
| hsa-miR-195 UAGCAGCACAGAAAUAUUGGC |
| hsa-miR-320a AAAAGCUGGGUUGAGAGGGCGA |
| hsa-miR-106b UAAAGUGCUGACAGUGCAGAU |
| hsa-miR-29c UAGCACCAUUUGAAAUCGGUUA |
| hsa-miR-219-2-3p AGAAUUGUGGCUGGACAUCUGU |
| hsa-miR-301a CAGUGCAAUAGUAUUGUCAAAGC |
| hsa-miR-296-3p GAGGGUUGGGUGGAGGCUCUCC |
| hsa-miR-30e UGUAAACAUCCUUGACUGGAAG |
| hsa-miR-365 UAAUGCCCCUAAAAAUCCUUAU |
| hsa-miR-374a UUAUAAUACAACCUGAUAAGUG |
| hsa-miR-340 UUAUAAAGCAAUGAGACUGAUU |
| hsa-miR-342-3p UCUCACACAGAAAUCGCACCCGU |
| hsa-miR-323-3p CACAUUACACGGUCGACCUCU |
| hsa-miR-326 CCUCUGGGCCCUUCCUCCAG |
| hsa-miR-151-3p CUAGACUGAAGCUCCUUGAGG |
| hsa-miR-148b UCAGUGCAUCACAGAACUUUGU |
| hsa-miR-331-3p GCCCCUGGGCCUAUCCUAGAA |
| hsa-miR-339-5p UCCCUGUCCUCCAGGAGCUCACG |
| hsa-miR-335 UCAAGAGCAAUAACGAAAAAUGU |
| ebv-miR-BHRF1-2 UAUCUUUUGCGGCAGAAAUUGA |
| ebv-miR-BHRF1-3 UAACGGGAAGUGUGUAAGCACA |
| ebv-miR-BART1-5p UCUUAGUGGAAGUGACGUGCUGUG |
| hsa-miR-422a ACUGGACUUAGGGUCAGAAGGC |
| hsa-miR-423-5p UGAGGGGCAGAGAGCGAGACUUU |
| hsa-miR-424 CAGCAGCAAUUCAUGUUUUGAA |
| hsa-miR-425 AAUGACACGAUCACUCCCGUUGA |
| hsa-miR-20b CAAAGUGCUCAUAGUGCAGGUAG |
| hcmv-miR-UL148D UCGUCCUCCCCUUCUUCACCG |
| hsa-miR-433 AUCAUGAUGGGCUCCUCGGUGU |
| kshv-miR-K12-1 AUUACAGGAAACUGGGUGUAAGC |
| kshv-miR-K12-2 AACUGUAGUCCGGGUCGAUCUG |
| kshv-miR-K12-3 UCACAUUCUGAGGACGGCAGCGA |
| hsa-miR-490-5p CCAUGGAUCUCCAGGUGGGU |
| hsa-miR-146b-5p UGAGAACUGAAUUCCAUAGGCU |
| hsa-miR-498 UUUCAAGCCAGGGGGCGUUUUUC |
| hsa-miR-525-5p CUCCAGAGGGAUGCACUUUCU |
| hsa-miR-516b AUCUGGAGGUAAGAAGCACUUU |
| hsa-miR-502-5p AUCCUUGCUAUCUGGGUGCUA |
| hsa-miR-508-5p UACUCCAGAGGGCGUCACUCAUG |
| hsa-miR-510 UACUCAGGAGAGUGGCAAUCAC |
| hsa-miR-553 AAAACGGUGAGAUUUUGUUUU |
| hsa-miR-92b UAUUGCACUCGUCCCGGCCUCC |
| hsa-miR-584 UUAUGGUUUGCCUGGGACUGAG |
| hsa-miR-586 UAUGCAUUGUAUUUUUAGGUCC |
| hsa-miR-548b-3p CAAGAACCUCAGUUGCUUUUGU |
| hsa-miR-596 AAGCCUGCCCGGCUCCUCGGG |
| hsa-miR-610 UGAGCUAAAUGUGUGCUGGGA |
| hsa-miR-612 GCUGGGCAGGGCUUCUGAGCUCCUU |
| hsa-miR-617 AGACUUCCCAUUUGAAGGUGGC |
| hsa-miR-622 ACAGUCUGCUGAGGUUGGAGC |
| hsa-miR-623 AUCCCUUGCAGGGGCUGUUGGGU |
| hsa-miR-630 AGUAUUCUGUACCAGGGAAGGU |
| hsa-miR-631 AGACCUGGCCCAGACCUCAGC |
| hsa-miR-638 AGGGAUCGCGGGCGGGUGGCGGCCU |
| hsa-miR-640 AUGAUCCAGGAACCUGCCUCU |
| hsa-miR-642 GUCCCUCUCCAAAUGUGUCUUG |
| hsa-miR-645 UCUAGGCUGGUACUGCUGA |
| hsa-miR-654-5p UGGUGGGCCGCAGAACAUGUGC |
| hsa-miR-657 GGCAGGUUCUCACCCUCUCUAGG |
| hsa-miR-542-5p UCGGGGAUCAUCAUGUCACGAGA |
| hcmv-miR-UL70-3p GGGGAUGGGCUGGCGCGCGG |
| ebv-miR-BART6-3p CGGGGAUCGGACUAGCCUUAGA |
| ebv-miR-BART6-3p CGGGGAUCGGACUAGCCUUAGA |
| ebv-miR-BART13 UGUAACUUGCCAGGGACGGCUGA |
| ebv-miR-BART16 UUAGAUAGAGUGGGUGUGUGCUCU |
| hsa-miR-300 UAUACAAGGGCAGACUCUCUCU |
| hsa-miR-374b AUAUAAUACAACCUGCUAAGUG |
| hsa-miR-935 CCAGUUACCGCUUCCGCUACCGC |
| hsa-miR-939 UGGGGAGCUGAGGCUCUGGGGGUG |
| hivl-miR-H1 CCAGGGAGGCGUGCCUGGGC |
| hsa-miR-720 UCUCGCUGGGGCCUCCA |
| hsa-miR-21* CAACACCAGUCGAUGGGCUGU |
| hsa-miR-22* AGUUCUUCAGUGGCAAGCUUUA |
| hsa-miR-25* AGGCGGAGACUUGGGCAAUUG |
| hsa-miR-200b* CAUCUUACUGGGCAGCAUUGGA |
| hsa-miR-30b* CUGGGAGGUGGAUGUUUACUUC |
| hsa-miR-135a* UAUAGGGAUUGGAGCCGUGGCG |
| hsa-miR-125b-2* UCACAAGUCAGGCUCUUGGGAC |
| hsa-miR-126* CAUUAUUACUUUUGGUACGCG |
| hsa-miR-149* AGGGAGGGACGGGGGCUGUGC |
| hsa-miR-194* CCAGUGGGGCUGCUGUUAUCUG |
| hsa-miR-30c-1* CUGGGAGAGGGUUGUUUACUCC |
| hsa-miR-30e* CUUUCAGUCGGAUGUUUACAGC |
| hsa-miR-377* AGAGGUUGCCCUUGGUGAAUUC |
| hsa-miR-340* UCCGUCUCAGUUACUUUAUAGC |
| hsa-miR-424* CAAAACGUGAGGCGCUGCUAU |
| hcmv-miR-US25-1* UCCGAACGCUAGGUCGGUUCUC |
| hsa-miR-519e* UUCUCCAAAAGGGAGCACUUUC |
| hsa-miR-92b* AGGGACGGGACGCGGUGCAGUG |
| ebv-miR-BART8* GUCACAAUCUAUGGGGUCGUAGA |

### OTHER EMBODIMENTS

While certain novel features of this invention shown and described are pointed out in the annexed claims, the invention is not intended to be limited to the details specified, since a person of ordinary skill in the relevant art will understand that various omissions, modifications, substitutions and changes in the forms and details of the invention illustrated and in its operation may be made without departing in any way from the present invention as defined in the claims. No feature of the invention is critical or essential unless it is expressly stated as being "critical" or "essential."

Those skilled in the art will recognize or be able to ascertain using no more than routine experimentation many equivalents to the specific embodiments of the invention described herein.

## Claims

1. A method for prognosing non-small cell lung cancer (NSCLC) relapse in a cancer patient comprising:
a) determining the level of expression of a first biomarker, hsa-miR-513b, in a sample from the cancer patient;
b) comparing the level of expression of said first biomarker in said sample to the level of expression of said first biomarker in a sample from a first reference patient having a history of a NSCLC diagnosis, wherein said first reference patient has been determined to have a good prognosis of no NSCLC relapse;
c) comparing the level of expression of said first biomarker in said sample to the level of expression of said first biomarker in a sample from a second reference patient having a history of a NSCLC diagnosis, wherein said second reference patient has been determined to have a poor prognosis of no NSCLC relapse; and
d) determining the prognosis of NSCLC relapse in said cancer patient, wherein:
i) a determination that the level of expression of said first biomarker in said sample from said cancer patient is similar to the level of expression of said first biomarker in said sample from said first reference patient indicates a good prognosis of no NSCLC relapse in said cancer patient, and
ii) a determination that the level of expression of said first biomarker in said sample from said cancer patient is similar to the level of expression of said first biomarker in said sample from said second reference patient indicates a poor prognosis of no NSCLC relapse in said cancer patient.

2. The method of claim 1, wherein said method further comprises:
a) determining the level of expression of at least one second biomarker selected from the group consisting of hsa-miR-650, hsa-miR-324-3p, and hsa-miR-1307 in said sample from said cancer patient,
b) comparing the level of expression of said second biomarker in said sample to the level of expression of said second biomarker in a sample from a first reference patient having a history of a NSCLC diagnosis, wherein said first reference patient has been determined to have a good prognosis of no NSCLC relapse;
c) comparing the level of expression of said second biomarker in said sample to the level of expression of said second biomarker in a sample from a second reference patient having a history of a NSCLC diagnosis, wherein said second reference patient has been determined to have a poor prognosis of no NSCLC relapse; and
d) determining the prognosis of NSCLC relapse in said cancer patient, wherein:
i) a determination that the level of expression of said second biomarker in said sample from said cancer patient is similar to the level of expression of said second biomarker in said sample from said first reference patient indicates a good prognosis of no NSCLC relapse in said cancer patient, and
ii) a determination that the level of expression of said second biomarker in said sample from said cancer patient is similar to the level of expression of said second biomarker in said sample from said second reference patient indicates a poor prognosis of no NSCLC relapse in said cancer patient.

3. The method of claim 1 or 2, wherein the sample is a tissue sample, particularly wherein said tissue sample is a tumor sample.

4. The method of any one of claims 1 to 3, wherein:
a) the prognosis occurs in said patient after a first cancer treatment;
b) the prognosis occurs in said patient prior to a first cancer treatment;
c) the prognosis occurs in said patient after a first cancer treatment, but before a second cancer treatment; or
d) the prognosis occurs in said patient after a second cancer treatment.

5. The method of claim 4, wherein said treatment comprises one or more of surgery, radiation therapy, and chemotherapy.

6. The method of any one of claims 1 to 5, wherein the level of expression of said first biomarker in said sample is determined by collecting nucleic acid molecules from said sample and, optionally, using a quantitative reverse transcription-polymerase chain reaction (qRT-PCR) to amplify said nucleic acid molecules to produce an amplified sample, particularly wherein said method comprises detecting said first biomarker in said amplified sample.

7. The method of claim 2, wherein the level of expression of said at least one second biomarker in said sample is determined by collecting nucleic acid molecules from said sample and, optionally, using a quantitative reverse transcription-polymerase chain reaction (qRT-PCR) to amplify said nucleic acid molecules to produce an amplified sample, particularly wherein said method comprises detecting said at least one second biomarker in said amplified sample.

8. The method of any one of claims 1 to 7, wherein said method comprises detecting the level of expression of said first biomarker using at least one single-stranded nucleic acid molecule that specifically hybridizes to said first biomarker, wherein said single-stranded nucleic acid molecule comprises at least 5, particularly at least 6, 7, 8, 10, 12, 15, 20, or 22, consecutive nucleotides of the sequence of hsa-miR-513b_st or its complement sequence.

9. The method of claim 2, wherein said method comprises detecting the level of expression of said at least one second biomarker using at least one single-stranded nucleic acid molecule that specifically hybridizes to said at least one second biomarker, wherein said single-stranded nucleic acid molecule comprises at least 5, particularly at least 6, 7, 8, 10, 12, 15, 20, or 22, consecutive nucleotides of the sequence of hsa-miR-650, hsa-miR-324-3p, or hsa-miR-1307 or its complement sequence.

10. The method of claim 8, wherein said method comprises detecting the level of expression of said first biomarker using a device to which said at least one single-stranded nucleic acid molecule is attached, particularly wherein said device is a microarray.

11. The method of claim 9, wherein said method comprises detecting the level of expression of said at least one second biomarker using a device to which said at least one single-stranded nucleic acid molecule is attached, particularly wherein said device is a microarray.

## Patentansprüche

1. Verfahren zum Prognostizieren des Rückfalls des nicht-kleinzelligen Lungenkrebs (NSCLC) in einem Krebspatienten, umfassend:
a) Bestimmen des Grades der Expression eines ersten Biomarkers, hsa-miR-513b, in einer Probe von dem Krebspatienten;
b) Vergleichen des Grades der Expression des ersten Biomarkers in der Probe mit dem Grad der Expression des ersten Biomarkers in einer Probe von einem ersten Referenzpatienten, der eine Vorgeschichte einer NSCLC-Diagnose hat, wobei für den ersten Referenzpatienten festgestellt wurde, dass er eine gute Prognose hat, keinen NSCLC-Rückfall zu haben;
c) Vergleichen des Grades der Expression des ersten Biomarkers in der Probe mit dem Grad der Expression des ersten Biomarkers in einer Probe von einem zweiten Referenzpatienten, der eine Vorgeschichte einer NSCLC-Diagnose hat, wobei für den zweiten Referenzpatienten festgestellt wurde, dass er eine schlechte Prognose hat, keinen NSCLC-Rückfall zu haben; und
d) Bestimmen der Prognose des Rückfalls von NSCLC in dem Krebspatienten, wobei:
i) eine Bestimmung, dass der Grad der Expression des ersten Biomarkers in der Probe von dem Krebspatienten ähnlich ist zum Grad der Expression des ersten Biomarkers in der Probe des ersten Referenzpatienten eine gute Prognose für keinen Rückfall von NSCLC in dem Krebspatienten anzeigt, und
ii) eine Bestimmung, dass der Grad der Expression des ersten Biomarkers in der Probe von dem Krebspatienten ähnlich ist zum Grad der Expression des ersten Biomarkers in der Probe von dem zweiten Referenzpatienten eine schlechte Prognose für keinen Rückfall von NSCLC in dem Krebspatienten anzeigt.

2. Verfahren nach Anspruch 1, wobei das Verfahren weiter umfasst:
a) Bestimmen des Grades der Expression von mindestens einem zweiten Biomarker, ausgewählt aus der Gruppe bestehend aus hsa-miR-650, hsa-miR-324-3p und hsa-miR-1307 in der Probe von dem Krebspatienten,
b) Vergleichen des Grades der Expression des zweiten Biomarkers in der Probe mit dem Grad der Expression des zweiten Biomarkers in einer Probe von einem ersten Referenzpatienten, der eine Vorgeschichte einer NSCLC-Diagnose hat, wobei für den ersten Referenzpatienten festgestellt wurde, dass er eine gute Prognose hat, keinen NSCLC-Rückfall zu haben;
c) Vergleichen des Grades der Expression des zweiten Biomarkers in der Probe mit dem Grad der Expression des zweiten Biomarkers in einer Probe von einem zweiten Referenzpatienten, der eine Vorgeschichte einer NSCLC-Diagnose hat, wobei für den zweiten Referenzpatienten festgestellt wurde, dass er eine schlechte Prognose hat, keinen NSCLC-Rückfall zu haben; und
d) Bestimmen der Prognose des Rückfalls von NSCLC in dem Krebspatienten, wobei:
i) eine Bestimmung, dass der Grad der Expression des zweiten Biomarkers in der Probe von dem Krebspatienten ähnlich ist zum Grad der Expression des zweiten Biomarkers in der Probe von dem ersten Referenzpatienten eine gute Prognose für keinen Rückfall von NSCLC in dem Krebspatienten anzeigt, und
ii) eine Bestimmung, dass der Grad der Expression des zweiten Biomarkers in der Probe von dem Krebspatienten ähnlich ist zum Grad der Expression des zweiten Biomarkers in der Probe von dem zweiten Referenzpatienten eine schlechte Prognose für keinen NSCLC-Rückfall in dem Krebspatienten anzeigt.

3. Verfahren nach Anspruch 1 oder 2, wobei die Probe eine Gewebeprobe ist, insbesondere wobei die Gewebeprobe eine Tumorprobe ist.

4. Verfahren nach einem beliebigen der Ansprüche 1 bis 3, wobei:
a) die Prognose in dem Krebspatienten nach einer ersten Krebsbehandlung auftritt;
b) die Prognose in dem Krebspatienten vor einer ersten Krebsbehandlung auftritt;
c) die Prognose in dem Krebspatienten nach einer ersten Krebsbehandlung aber vor einer zweiten Krebsbehandlung auftritt; oder
d) die Prognose in dem Krebspatienten nach einer zweiten Krebsbehandlung auftritt.

5. Verfahren nach Anspruch 4, wobei die Behandlung eine(s) oder mehrere von Operation, Bestrahlungstherapie und Chemotherapie umfasst.

6. Verfahren nach einem beliebigen der Ansprüche 1 bis 5, wobei der Grad der Expression des ersten Biomarkers in der Probe bestimmt wird durch Sammeln von Nukleinsäuremolekülen aus der Probe und optional Verwenden einer quantitativen reversen Transkriptionspolymerasekettenreaktion (qRT-PCR) um die Nukleinsäuremoleküle zu amplifizieren, um eine amplifizierte Probe herzustellen, insbesondere wobei das Verfahren das Detektieren des ersten Biomarkers in der amplifizierten Probe umfasst.

7. Verfahren nach Anspruch 2, wobei der Grad der Expression des mindestens einen zweiten Biomarkers in der Probe bestimmt wird durch Sammeln von Nukleinsäuremolekülen aus der Probe und optional Verwenden einer quantitativen reversen Transkriptionspolymerasekettenreaktion (qRT-PCR) um die Nukleinsäuremoleküle zu amplifizieren, um eine amplifizierte Probe herzustellen, insbesondere wobei das Verfahren das Detektieren des mindestens einen zweiten Biomarkers in der amplifizierten Probe umfasst.

8. Verfahren nach einem beliebigen der Ansprüche 1 bis 7, wobei das Verfahren das Detektieren des Grades der Expression des ersten Biomarkers unter Verwendung mindestens eines einzelsträngigen Nukleinsäuremoleküls, das spezifisch an den ersten Biomarker hybridisiert, umfasst, wobei das einzelsträngige Nukleinsäuremolekül mindestens 5, insbesondere mindestens 6, 7, 8, 10, 12, 15, 20 oder 22 aufeinanderfolgende Nukleotide der Sequenz von hsa-miR-513b_st oder dessen/ihrer komplementärer Sequenz umfasst.

9. Verfahren nach Anspruch 2, wobei das Verfahren das Detektieren des Grades der Expression des mindestens einen zweiten Biomarkers unter Verwendung mindestens eines einzelsträngigen Nukleinsäuremoleküls, das spezifisch an den mindestens einen zweiten Biomarker hybridisiert, umfasst, wobei das einzelsträngige Nukleinsäuremolekül mindestens 5, insbesondere mindestens 6, 7, 8, 10, 12, 15, 20 oder 22 aufeinanderfolgende Nukleotide der Sequenz von hsa-miR-650, hsa-miR-324-3p oder hsa-miR-1307 oder dessen/ihrer komplementärer Sequenz umfasst.

10. Verfahren nach Anspruch 8, wobei das Verfahren das Detektieren des Grades der Expression des ersten Biomarkers unter Verwendung einer Vorrichtung umfasst, an die das mindestens eine einzelsträngige Nukleinsäuremolekül angeheftet ist, insbesondere wobei die Vorrichtung ein Microarray ist.

11. Verfahren nach Anspruch 9, wobei das Verfahren das Detektieren des Grades der Expression des mindestens einen zweiten Biomarkers unter Verwendung einer Vorrichtung umfasst, an die das mindestens eine einzelsträngige Nukleinsäuremolekül angeheftet ist, insbesondere wobei die Vorrichtung ein Microarray ist.

## Revendications

1. Procédé de pronostic d'une rechute du cancer du poumon non à petites cellules (CPNPC) chez un patient cancéreux comprenant :
a) la détermination du taux d'expression d'un premier biomarqueur, hsa-miR-513b, dans un échantillon du patient cancéreux ;
b) la comparaison du taux d'expression dudit premier biomarqueur dans ledit échantillon au taux d'expression dudit premier biomarqueur dans un échantillon d'un premier patient de référence ayant des antécédents de diagnostic du CPNPC, où il a été déterminé que ledit premier patient de référence présentait un bon pronostic d'absence de rechute du CPNPC ;
c) la comparaison du taux d'expression dudit premier biomarqueur dans ledit échantillon au taux d'expression dudit premier biomarqueur dans un échantillon d'un second patient de référence ayant des antécédents de diagnostic du CPNPC, où il a été déterminé que ledit second patient de référence présentait un mauvais pronostic d'absence de rechute du CPNPC ; et
d) la détermination du pronostic de rechute du CPNPC chez ledit patient cancéreux, où :
i) une détermination que le taux d'expression dudit premier biomarqueur dans ledit échantillon dudit patient cancéreux est similaire au taux d'expression dudit premier biomarqueur dans ledit échantillon dudit premier patient de référence indique un bon pronostic d'absence de rechute du CPNPC chez ledit patient cancéreux, et
ii) une détermination que le taux d'expression dudit premier biomarqueur dans ledit échantillon dudit patient cancéreux est similaire au taux d'expression dudit premier biomarqueur dans ledit échantillon dudit second patient de référence indique un mauvais pronostic d'absence de rechute du CPNPC chez ledit patient cancéreux.

2. Procédé selon la revendication 1, où ledit procédé comprend en outre :
a) la détermination du taux d'expression d'au moins un second biomarqueur sélectionné dans le groupe consistant en hsa-miR-650, hsa-miR-324-3p, et hsa-miR-1307 dans ledit échantillon dudit patient cancéreux,
b) la comparaison du taux d'expression dudit second biomarqueur dans ledit échantillon au taux d'expression dudit second biomarqueur dans un échantillon d'un premier patient de référence ayant des antécédents de diagnostic du CPNPC, où il a été déterminé que ledit premier patient de référence présentait un bon pronostic d'absence de rechute du CPNPC ;
c) la comparaison du taux d'expression dudit second biomarqueur dans ledit échantillon au taux d'expression dudit second biomarqueur dans un échantillon d'un second patient de référence ayant des antécédents de diagnostic du CPNPC, où il a été déterminé que ledit second patient de référence présentait un mauvais pronostic d'absence de rechute du CPNPC ; et
d) la détermination du pronostic de rechute du CPNPC chez ledit patient cancéreux, où :
i) une détermination que le taux d'expression dudit second biomarqueur dans ledit échantillon dudit patient cancéreux est similaire au taux d'expression dudit second biomarqueur dans ledit échantillon dudit premier patient de référence indique un bon pronostic d'absence de rechute du CPNPC chez ledit patient cancéreux, et
ii) une détermination que le taux d'expression dudit second biomarqueur dans ledit échantillon dudit patient cancéreux est similaire au taux d'expression dudit second biomarqueur dans ledit échantillon dudit second patient de référence indique un mauvais pronostic d'absence de rechute du CPNPC chez ledit patient cancéreux.

3. Procédé selon la revendication 1 ou 2, dans lequel l'échantillon est un échantillon de tissu, particulièrement dans lequel ledit échantillon de tissu est un échantillon tumoral.

4. Procédé selon l'une quelconque des revendications 1 à 3, dans lequel :
a) le pronostic est établi chez ledit patient après un premier traitement du cancer ;
b) le pronostic est établi chez ledit patient avant un premier traitement du cancer ;
c) le pronostic est établi chez ledit patient après un premier traitement du cancer, mais avant un second traitement du cancer ; ou
d) le pronostic est établi chez ledit patient après un second traitement du cancer.

5. Procédé selon la revendication 4, dans lequel ledit traitement comprend un ou plusieurs parmi la chirurgie, la radiothérapie, et la chimiothérapie.

6. Procédé selon l'une quelconque des revendications 1 à 5, dans lequel le taux d'expression dudit premier biomarqueur dans ledit échantillon est déterminé en collectant des molécules d'acide nucléique dudit échantillon et, facultativement, en utilisant une transcription inverse-réaction en chaîne par polymérase quantitative (qRT-PCR) pour amplifier lesdites molécules d'acide nucléique afin de produire un échantillon amplifié, particulièrement où ledit procédé comprend la détection dudit premier biomarqueur dans ledit échantillon amplifié.

7. Procédé selon la revendication 2, dans lequel le taux d'expression dudit au moins un second biomarqueur dans ledit échantillon est déterminé en collectant des molécules d'acide nucléique dudit échantillon et, facultativement, en utilisant une transcription inverse-réaction en chaîne par polymérase quantitative (qRT-PCR) pour amplifier lesdites molécules d'acide nucléique afin de produire un échantillon amplifié, particulièrement où ledit procédé comprend la détection dudit au moins un second biomarqueur dans ledit échantillon amplifié.

8. Procédé selon l'une quelconque des revendications 1 à 7, où ledit procédé comprend la détection du taux d'expression dudit premier biomarqueur en utilisant au moins une molécule d'acide nucléique simple brin qui s'hybride spécifiquement audit premier biomarqueur, où ladite molécule d'acide nucléique simple brin comprend au moins 5, particulièrement au moins 6, 7, 8, 10, 12, 15, 20, ou 22 nucléotides consécutifs de la séquence de hsa-miR-513b_st ou sa séquence complémentaire.

9. Procédé selon la revendication 2, où ledit procédé comprend la détection du taux d'expression dudit au moins un second biomarqueur en utilisant au moins une molécule d'acide nucléique simple brin qui s'hybride spécifiquement audit au moins un second biomarqueur, où ladite molécule d'acide nucléique simple brin comprend au moins 5, particulièrement au moins 6, 7, 8, 10, 12, 15, 20, ou 22 nucléotides consécutifs de la séquence de hsa-miR-650, hsa-miR-324-3p, ou hsa-miR-1307 ou sa séquence complémentaire.

10. Procédé selon la revendication 8, où ledit procédé comprend la détection du taux d'expression dudit premier biomarqueur en utilisant un dispositif auquel ladite au moins une molécule d'acide nucléique simple brin est attachée, particulièrement dans lequel ledit dispositif est un microréseau.

11. Procédé selon la revendication 9, où ledit procédé comprend la détection du taux d'expression dudit au moins un second biomarqueur en utilisant un dispositif auquel ladite au moins une molécule d'acide nucléique simple brin est attachée, particulièrement dans lequel ledit dispositif est un microréseau.
